# EUROPEAN PATENT SPECIFICATION

(11) **EP 3 126 349 B1**
(45) Date of publication and mention of the grant of the patent: **26.06.2019**
(21) Application number: 15712968.5
(22) Date of filing: 01.04.2015
(51) Int. Cl.: C07D 401/14, C07D 405/14, C07D 413/14, C07D 213/81, C07D 401/04, C07D 413/12, C07D 417/06, C07D 417/12, C07D 491/10, A61K 31/4439, A61P 29/00

(54) **5,6-DISUBSTITUTED PYRIDINE-2-CARBOXAMIDES AS CANNABINOID RECEPTOR AGONISTS**
5,6-DISUBSTITUIERTE PYRIDIN-2-CARBOXAMIDE ALS CANNABINOID-REZEPTOR-AGONISTEN
PYRIDINE-2-CARBOXAMIDES BISUBSTITUÉS EN 5,6 SERVANT D'AGONISTES DU RÉCEPTEUR CANNABINOÏDE

(30) Priority: 04.04.2014 EP 14163554
(43) Date of publication of application: 08.02.2017
(73) Proprietor: F. Hoffmann-La Roche AG, 4070 Basel (CH); Eidgenoessische Technische Hochschule Zuerich, 8092 Zürich (CH)
(72) Inventor: GOBBI, Luca, CH-4463 Buus (CH); GRETHER, Uwe, 79588 Efringen-Kirchen (DE); NETTEKOVEN, Matthias, 79639 Grenzach-Wyhlen (DE); ROEVER, Stephan, 79594 Inzlingen (DE); ROGERS-EVANS, Mark, CH-4103 Bottmingen (CH); SLAVIK, Roger, CH-8400 Winterthur (CH)
(74) Representative: Pomeranc, Didier
(86) International application number: PCT/EP2015/057144
(87) International publication number: WO 2015/150438

(56) References cited:
- US-A1- 2012 065 212
- US-A1- 2012 316 147
- US-A1- 2013 109 665
- MUCCIOLI G G ET AL: "Latest advances in cannabinoid receptor antagonists and inverse agonists", EXPERT OPINION ON THERAPEUTIC PATENTS, INFORMA HEALTHCARE, GB, vol. 16, no. 10, 1 January 2006 (2006-01-01), pages 1405-1423, XP002407338, ISSN: 1354-3776, DOI: 10.1517/13543776.16.10.1405

## Description

The present invention relates to organic compounds useful for therapy and/or prophylaxis in a mammal, and in particular to compounds that are preferential inverse agonists of the Cannabinoid Receptor 2.

The present invention relates in particular to a compound of formula (I) 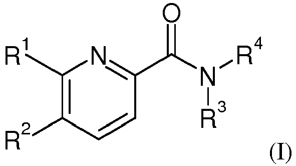 wherein
R¹ is alkylsulfanyl, cycloalkylalkoxy, halophenyl or halogen;
R² is phenyl, halophenyl, cycloalkylalkoxy, alkoxyazetidinyl, 2-oxa-6-azaspiro[3.3]heptyl, (haloalkyl)(hydroxy)pyrrolidinyl, halo-5-azaspiro[2.4]heptyl, (alkyl)(halo)azetidinyl or (cycloalkyl)(halo)azetidinyl;
one of R³ and R⁴ is hydrogen and the other one is -(CR⁵R⁶)ₘ-(CH₂)ₙ-R⁷;
or R³ and R⁴ together with the nitrogen atom to which they are attached form aminocarbonyl-dioxo-thiazolidinyl or (aminocarbonyl)(halo)pyrrolidinyl;
R⁵ and R⁶ are independently selected from hydrogen, alkyl, cycloalkylalkyl and alkylsulfanylalkyl;
or R⁵ and R⁶ together with the carbon atom to which they are attached form oxetanyl;
R⁷ is alkoxycarbonyl, oxazol-2-yl, 5-alkyl-1,2,4-oxadiazol-3-yl, aminocarbonyl, alkylaminocarbonyl, thiazol-2-yl, 5-halophenyl-1,3,4-oxadiazol-2-yl or hydroxycycloalkyl, haloalkylaminocarbonyl or haloazetidinylcarbonyl;
m is 0 or 1;
n is 0 or 1;
or a pharmaceutically acceptable salt or ester thereof.

The compound of formula (I) is particularly useful in the treatment or prophylaxis of pain, neuropathic pain, asthma, osteoporosis, inflammation, psychiatric diseases, psychosis, oncology, encephalitis, malaria, allergy, immunological disorders, arthritis, gastrointestinal disorders, psychiatric disorders rheumatoid arthritis, psychosis and allergy.

The cannabinoid receptors are a class of cell membrane receptors belonging to the G protein-coupled receptor superfamily. There are currently two known subtypes, termed Cannabinoid Receptor 1 (CB1) and Cannabinoid Receptor 2 (CB2). The CB1 receptor is mainly expressed in the central nervous (i.e. amygdala cerebellum, hippocampus) system and to a lesser amount in the periphery. CB2, which is encoded by the CNR2 gene, is mostly expressed peripherally, on cells of the immune system, such as macrophages and T-cells (Ashton, J. C. et al. Curr Neuropharmacol 2007, 5(2), 73-80; Miller, A. M. et al. Br J Pharmacol 2008, 153(2), 299-308; Centonze, D., et al. Curr Pharm Des 2008, 14(23), 2370-42), and in the gastrointestinal system (Wright, K. L. et al. Br J Pharmacol 2008, 153(2), 263-70). The CB2 receptor is also widely distributed in the brain where it is found primarily on microglia and not neurons (Cabral, G. A. et al. Br J Pharmacol 2008, 153(2): 240-51).

The interest in CB2 receptor ligands has been steadily on the rise during the last decade (currently 30-40 patent applications/year). Evidence from different sources support the view that lipid endocannabinoid signaling through CB2 receptors represents an aspect of the mammalian protective armamentarium (Pacher, P. Prog Lipid Res 2011, 50, 193). Its modulation by either selective CB2 receptor agonists or inverse agonists/antagonists (depending on the disease and its stage) holds unique therapeutic potential in a huge number of diseases. For CB2 inverse agonists/antagonists therapeutic opportunities have been demonstrated for many pathological conditions including pain (Pasquini, S. J Med Chem 2012, 55(11): 5391), neuropathic pain (Garcia-Gutierrez, M.S. Br J Pharmacol 2012, 165(4): 951), psychiatric disorders (Garcia-Gutierrez, M.S. Br J Pharmacol 2012, 165(4): 951), psychosis (Garcia-Gutierrez, M.S. Br J Pharmacol 2012, 165(4): 951), osteoporosis and inflammation (Sophocleous, A. Calcif Tissue Int 2008, 82(Suppl. 1):Abst OC18), psychiatric diseases and psychosis (Garcia-Gutierrez, M.S. Br J Pharmacol 2012, 165(4): 951), oncology (Preet, A. Cancer Prev Res 2011, 4: 65), encephalitis and malaria (Zimmer, A. WO 2011045068), allergy and inflammation (Ueda, Y. Life Sci 2007, 80(5): 414), encephalitis and malaria (Zimmer, WO 2011045068), asthma (Lunn, C.A. J Pharmacol Exp Ther 2006, 316(2): 780), immunological disorders (Fakhfouri, G. Neuropharmacology 2012, 63(4): 653), rheumatoid arthritis (Chackalamannil, S. US 7776889), arthritis (Lunn, C.A. J Pharmacol Exp Ther 2006, 316(2): 780), and gastrointestinal disorders (Barth, F. FR 2887550). Further background information can be found in US 2012/316147, US 2012/065212 and Muccioli et al. Expert Opinion on Therapeutic Patents, 2006, 16, 1405-1423.

The compounds of the invention bind to and modulate the CB2 receptor and have lower CB1 receptor activity.

In the present description the term "alkyl", alone or in combination, signifies a straight-chain or branched-chain alkyl group with 1 to 8 carbon atoms, particularly a straight or branched-chain alkyl group with 1 to 6 carbon atoms and more particularly a straight or branched-chain alkyl group with 1 to 4 carbon atoms. Examples of straight-chain and branched-chain C1-C8 alkyl groups are methyl, ethyl, propyl, isopropyl, butyl, isobutyl, tert.-butyl, the isomeric pentyls, the isomeric hexyls, the isomeric heptyls and the isomeric octyls, particularly methyl, ethyl, propyl, butyl and pentyl. Particular examples of alkyl are methyl, ethyl, isopropyl, butyl, isobutyl, tert.-butyl and pentyl. Methyl, ethyl and isobutyl are particular examples of alkyl in the compound of formula (I).

The term "cycloalkyl", alone or in combination, signifies a cycloalkyl ring with 3 to 8 carbon atoms and particularly a cycloalkyl ring with 3 to 6 carbon atoms. Examples of cycloalkyl are cyclopropyl, cyclobutyl, cyclopentyl and cyclohexyl, cycloheptyl and cyclooctyl. Particular examples of "cycloalkyl" are cyclopropyl and cyclohexyl.

The term "alkoxy", alone or in combination, signifies a group of the formula alkyl-O- in which the term "alkyl" has the previously given significance, such as methoxy, ethoxy, n-propoxy, isopropoxy, n-butoxy, isobutoxy, sec-butoxy and tert.-butoxy. Particular "alkoxy" are methoxy and ethoxy.

The term "oxy", alone or in combination, signifies the -O- group.

The term "oxo", alone or in combination, signifies the =O group.

The terms "halogen" or "halo", alone or in combination, signifies fluorine, chlorine, bromine or iodine and particularly fluorine, chlorine or bromine, more particularly fluorine and chlorine. The term "halo", in combination with another group, denotes the substitution of said group with at least one halogen, particularly substituted with one to five halogens, particularly one to four halogens, i.e. one, two, three or four halogens.

The terms "hydroxyl" and "hydroxy", alone or in combination, signify the -OH group.

The term "carbonyl", alone or in combination, signifies the -C(O)- group.

The term "amino", alone or in combination, signifies the primary amino group (-NH2), the secondary amino group (-NH-), or the tertiary amino group (-N-).

The term "aminocarbonyl", alone or in combination, signifies the -C(O)-NH₂, -C(O)-NH- or -C(O)-N- group.

The term "sulfanyl", alone or in combination, signifies the -S- group.

The term "pharmaceutically acceptable salts" refers to those salts which retain the biological effectiveness and properties of the free bases or free acids, which are not biologically or otherwise undesirable. The salts are formed with inorganic acids such as hydrochloric acid, hydrobromic acid, sulfuric acid, nitric acid, phosphoric acid, particularly hydrochloric acid, and organic acids such as acetic acid, propionic acid, glycolic acid, pyruvic acid, oxalic acid, maleic acid, malonic acid, succinic acid, fumaric acid, tartaric acid, citric acid, benzoic acid, cinnamic acid, mandelic acid, methanesulfonic acid, ethanesulfonic acid, p-toluenesulfonic acid, salicylic acid, N-acetylcystein. In addition these salts may be prepared form addition of an inorganic base or an organic base to the free acid. Salts derived from an inorganic base include, but are not limited to, the sodium, potassium, lithium, ammonium, calcium, magnesium salts. Salts derived from organic bases include, but are not limited to salts of primary, secondary, and tertiary amines, substituted amines including naturally occurring substituted amines, cyclic amines and basic ion exchange resins, such as isopropylamine, trimethylamine, diethylamine, triethylamine, tripropylamine, ethanolamine, lysine, arginine, N-ethylpiperidine, piperidine, polyamine resins. The compound of formula (I) can also be present in the form of zwitterions. Particularly preferred pharmaceutically acceptable salts of compounds of formula (I) are the salts of hydrochloric acid, hydrobromic acid, sulfuric acid, phosphoric acid and methanesulfonic acid.

"Pharmaceutically acceptable esters" means that the compound of general formula (I) may be derivatised at functional groups to provide derivatives which are capable of conversion back to the parent compounds in vivo. Examples of such compounds include physiologically acceptable and metabolically labile ester derivatives, such as methoxymethyl esters, methylthiomethyl esters and pivaloyloxymethyl esters.

If one of the starting materials or compounds of formula (I) contain one or more functional groups which are not stable or are reactive under the reaction conditions of one or more reaction steps, appropriate protecting groups (as described e.g. in "Protective Groups in Organic Chemistry" by T. W. Greene and P. G. M. Wuts, 3rd Ed., 1999, Wiley, New York) can be introduced before the critical step applying methods well known in the art. Such protecting groups can be removed at a later stage of the synthesis using standard methods described in the literature. Examples of protecting groups are tert-butoxycarbonyl (Boc), 9-fluorenylmethyl carbamate (Fmoc), 2-trimethylsilylethyl carbamate (Teoc), carbobenzyloxy (Cbz) and p-methoxybenzyloxycarbonyl (Moz).

The compound of formula (I) can contain several asymmetric centers and can be present in the form of optically pure enantiomers, mixtures of enantiomers such as, for example, racemates, mixtures of diastereo¬isomers, diastereoisomeric racemates or mixtures of diastereoisomeric racemates.

The term "asymmetric carbon atom" means a carbon atom with four different substituents. According to the Cahn-Ingold-Prelog Convention an asymmetric carbon atom can be of the "R" or "S" configuration.

The invention relates in particular to:
A compound of formula (I) wherein R¹ is cycloalkylalkoxy or halophenyl;
A compound of formula (I) wherein R¹ is cyclopropylmethoxy or chlorophenyl;
A compound of formula (I) wherein R² is phenyl, halophenyl, cycloalkylalkoxy or alkoxyazetidinyl;
A compound of formula (I) wherein R² is phenyl, chlorophenyl, cyclopropylmethoxy or methoxyazetidinyl;
A compound of formula (I) wherein R⁵ and R⁶ are independently selected from hydrogen and alkyl;
A compound of formula (I) wherein R⁵ and R⁶ are independently selected from hydrogen, methyl, ethyl and isobutyl;
A compound of formula (I) wherein R⁷ is alkoxycarbonyl, oxazol-2-yl, thiazol-2-yl or aminocarbonyl; and
A compound of formula (I) wherein R⁷ is methoxycarbonyl, oxazol-2-yl, thiazol-2-yl or aminocarbonyl.

The invention further relates to a compound or formula (I) selected from:
Methyl 2-[[5-(4-chlorophenyl)-6-methylsulfanylpyridine-2-carbonyl]amino]-2-methylpropanoate;
Methyl 2-[[5-(4-chlorophenyl)-6-(cyclopropylmethoxy)pyridine-2-carbonyl] amino]-2-methylpropanoate;
5-(4-Chlorophenyl)-6-(cyclopropylmethoxy)-*N*-[2-(1,3-oxazol-2-yl)propan-2-yl]pyridine-2-carboxamide;
5-(4-Chlorophenyl)-6-(cyclopropylmethoxy)-*N*-[2-(5-methyl-1,2,4-oxadiazol-3-yl)propan-2-yl]pyridine-2-carboxamide;
6-(Cyclopropylmethoxy)-*N*-[2-(1,3-oxazol-2-yl)propan-2-yl]-5-phenylpyridine-2-carboxamide;
6-(Cyclopropylmethoxy)-*N*-[3-(methylcarbamoyl)pentan-3-yl]-5-phenylpyridine-2-carboxamide;
6-(3-Chlorophenyl)-5-(cyclopropylmethoxy)-*N*-[3-(methylcarbamoyl)pentan-3-yl]pyridine-2-carboxamide;
6-(Cyclopropylmethoxy)-5-(3-methoxyazetidin-1-yl)-*N*-[2-(5-methyl-1,2,4-oxadiazol-3-yl)propan-2-yl]pyridine-2-carboxamide;
6-(3-Chlorophenyl)-5-(cyclopropylmethoxy)-*N*-[2-(5-methyl-1,2,4-oxadiazol-3-yl)propan-2-yl]pyridine-2-carboxamide;
*N*-[(2*S*)-1-amino-3-cyclopropyl-1-oxopropan-2-yl]-6-(cyclopropylmethoxy)-5-(3-methoxyazetidin-1-yl)pyridine-2-carboxamide;
6-(Cyclopropylmethoxy)-5-(2-oxa-6-azaspiro[3.3]heptan-6-yl)-*N*-[2-(1,3-thiazol-2-yl)propan-2-yl]pyridine-2-carboxamide;
*N*-[(2*S*)-1-amino-4-methyl-1-oxopentan-2-yl]-6-(cyclopropylmethoxy)-5-(2-oxa-6-azaspiro[3.3]heptan-6-yl)pyridine-2-carboxamide;
6-(3-Chlorophenyl)-5-(cyclopropylmethoxy)-*N*-[2-(1,3-thiazol-2-yl)propan-2-yl]pyridine-2-carboxamide;
*N*-[(2*S*)-1-amino-3-cyclopropyl-1-oxopropan-2-yl]-5,6-bis(cyclopropylmethoxy)pyridine-2-carboxamide;
*N*-[(2*S*)-1-amino-4-methyl-1-oxopentan-2-yl]-6-(cyclopropylmethoxy)-5-(3-methoxyazetidin-1-yl)pyridine-2-carboxamide;
*N*-[(2*S*)-1-amino-4-methylsulfanyl-1-oxobutan-2-yl]-6-chloro-5-(cyclopropylmethoxy)pyridine-2-carboxamide;
6-Chloro-5-(cyclopropylmethoxy)-*N*-[3-(methylcarbamoyl)pentan-3-yl]pyridine-2-carboxamide;
6-Chloro-5-(cyclopropylmethoxy)-*N*-[(1*S*)-2-cyclopropyl-1-(5-methyl-1,2,4-oxadiazol-3-yl)ethyl]pyridine-2-carboxamide;
3-[6-(Cyclopropylmethoxy)-5-(3-methoxyazetidin-1-yl)pyridine-2-carbonyl]-1,1-dioxo-1,3-thiazolidine-4-carboxamide;
Ethyl 2-[[6-(cyclopropylmethoxy)-5-(3-methoxyazetidin-1-yl)pyridine-2-carbonyl]amino]-2-ethylbutanoate;
6-(Cyclopropylmethoxy)-*N*-[1-[5-(4-fluorophenyl)-1,3,4-oxadiazol-2-yl]-2-methylpropan-2-yl]-5-(3-methoxyazetidin-1-yl)pyridine-2-carboxamide;
*N*-[3-(2-Amino-2-oxoethyl)oxetan-3-yl]-6-(cyclopropylmethoxy)-5-(3-methoxyazetidin-1-yl)pyridine-2-carboxamide;
6-(Cyclopropylmethoxy)-5-(3-methoxyazetidin-1-yl)-*N*-[3-(methylcarbamoyl)pentan-3-yl]pyridine-2-carboxamide;
6-(Cyclopropylmethoxy)-*N*-[(1-hydroxycyclohexyl)methyl]-5-(3-methoxyazetidin-1-yl)pyridine-2-carboxamide;
6-(Cyclopropylmethoxy)-N-[3-(2-fluoroethylcarbamoyl)pentan-3-yl]-5-(3-methoxyazetidin-1-yl)pyridine-2-carboxamide;
6-(Cyclopropylmethoxy)-N-[3-(3-fluoroazetidine-1-carbonyl)pentan-3-yl]-5-(3-methoxyazetidin-1-yl)pyridine-2-carboxamide;
6-(Cyclopropylmethoxy)-N-[3-(3-fluoropropylcarbamoyl)pentan-3-yl]-5-(3-methoxyazetidin-1-yl)pyridine-2-carboxamide;
(2S)-1-[6-(Cyclopropylmethoxy)-5-[3-hydroxy-3-(trifluoromethyl)pyrrolidin-1-yl]pyridine-2-carbonyl]-4,4-difluoropyrrolidine-2-carboxamide;
N-[(2S)-1-Amino-4-methyl-1-oxopentan-2-yl]-6-(cyclopropylmethoxy)-5-[3-hydroxy-3-(trifluoromethyl)pyrrolidin-1-yl]pyridine-2-carboxamide;
N-[(2S)-1-Amino-4-methyl-1-oxopentan-2-yl]-6-(cyclopropylmethoxy)-5-(2,2-difluoro-5-azaspiro[2.4]heptan-5-yl)pyridine-2-carboxamide;
(2S)-1-[6-(Cyclopropylmethoxy)-5-(2,2-difluoro-5-azaspiro[2.4]heptan-5-yl)pyridine-2-carbonyl]-4,4-difluoropyrrolidine-2-carboxamide;
N-[3-(2-Amino-2-oxoethyl)oxetan-3-yl]-6-(cyclopropylmethoxy)-5-(2,2-difluoro-5-azaspiro[2.4]heptan-5-yl)pyridine-2-carboxamide;
N-[(2S)-1-Amino-4-methyl-1-oxopentan-2-yl]-6-(cyclopropylmethoxy)-5-(3-fluoro-3-methylazetidin-1-yl)pyridine-2-carboxamide;
(2S)-1-[6-(Cyclopropylmethoxy)-5-(3-fluoro-3-methylazetidin-1-yl)pyridine-2-carbonyl]-4,4-difluoropyrrolidine-2-carboxamide;
N-[3-(2-Amino-2-oxoethyl)oxetan-3-yl]-6-(cyclopropylmethoxy)-5-(3-fluoro-3-methylazetidin-1-yl)pyridine-2-carboxamide;
N-[(2S)-1-Amino-4-methyl-1-oxopentan-2-yl]-5-(3-cyclopropyl-3-fluoroazetidin-1-yl)-6-(cyclopropylmethoxy)pyridine-2-carboxamide;
(2S)-1-[5-(3-Cyclopropyl-3-fluoroazetidin-1-yl)-6-(cyclopropylmethoxy)pyridine-2-carbonyl]-4,4-difluoropyrrolidine-2-carboxamide;
N-[3-(2-Amino-2-oxoethyl)oxetan-3-yl]-5-(3-cyclopropyl-3-fluoroazetidin-1-yl)-6-(cyclopropylmethoxy)pyridine-2-carboxamide;
(2S)-1-[6-(4-Chlorophenyl)-5-(cyclopropylmethoxy)pyridine-2-carbonyl]-4,4-difluoropyrrolidine-2-carboxamide;
6-(4-Chlorophenyl)-5-(cyclopropylmethoxy)-N-[2-(5-methyl-1,2,4-oxadiazol-3-yl)propan-2-yl]pyridine-2-carboxamide;
5-(3-Cyclopropyl-3-fluoroazetidin-1-yl)-6-(cyclopropylmethoxy)-N-[3-(3-fluoropropylcarbamoyl)pentan-3-yl]pyridine-2-carboxamide; and
N-[(2S)-1-Amino-4-methyl-1-oxopentan-2-yl]-6-(4-chlorophenyl)-5-(cyclopropylmethoxy)pyridine-2-carboxamide.

The invention further relates in particular to a compound of formula (I) selected from
Methyl 2-[[5-(4-chlorophenyl)-6-(cyclopropylmethoxy)pyridine-2-carbonyl] amino]-2-methylpropanoate;
6-(Cyclopropylmethoxy)-N-[2-(1,3-oxazol-2-yl)propan-2-yl]-5-phenylpyridine-2-carboxamide;
6-(3-Chlorophenyl)-5-(cyclopropylmethoxy)-N-[2-(1,3-thiazol-2-yl)propan-2-yl]pyridine-2-carboxamide;
N-[(2S)-1-amino-4-methyl-1-oxopentan-2-yl]-6-(cyclopropylmethoxy)-5-(3-methoxyazetidin-1-yl)pyridine-2-carboxamide;
3-[6-(Cyclopropylmethoxy)-5-(3-methoxyazetidin-1-yl)pyridine-2-carbonyl]-1,1-dioxo-1,3-thiazolidine-4-carboxamide;
Ethyl 2-[[6-(cyclopropylmethoxy)-5-(3-methoxyazetidin-1-yl)pyridine-2-carbonyl]amino]-2-ethylbutanoate;
N-[3-(2-Amino-2-oxoethyl)oxetan-3-yl]-6-(cyclopropylmethoxy)-5-(3-methoxyazetidin-1-yl)pyridine-2-carboxamide;
6-(Cyclopropylmethoxy)-5-(3-methoxyazetidin-1-yl)-N-[3-(methylcarbamoyl)pentan-3-yl]pyridine-2-carboxamide; and
N-[(2S)-1-Amino-4-methyl-1-oxopentan-2-yl]-6-(4-chlorophenyl)-5-(cyclopropylmethoxy)pyridine-2-carboxamide.

Unless indicated otherwise, R¹-R⁷ have in the following schemes the meaning as defined above.

The synthesis of the compounds with the general structure **I** can, for example, be accomplished according to the following schemes.

Following the procedure according to scheme 1, compound **AA** (X = Cl, Br, I or trifluoromethanesulfonate; R' = H, methyl, ethyl, isopropyl, tert. butyl or another suitable protecting group described for example in T.W. Greene et al., Protective Groups in Organic Chemistry, John Wiley and Sons Inc. New York 1999, 3rd edition) can be used as starting material. **AA** is either commercially available, described in the literature, can be synthesized by a person skilled in the art, can be synthesized as described in schemes 3 and 6 or as described in the experimental part.

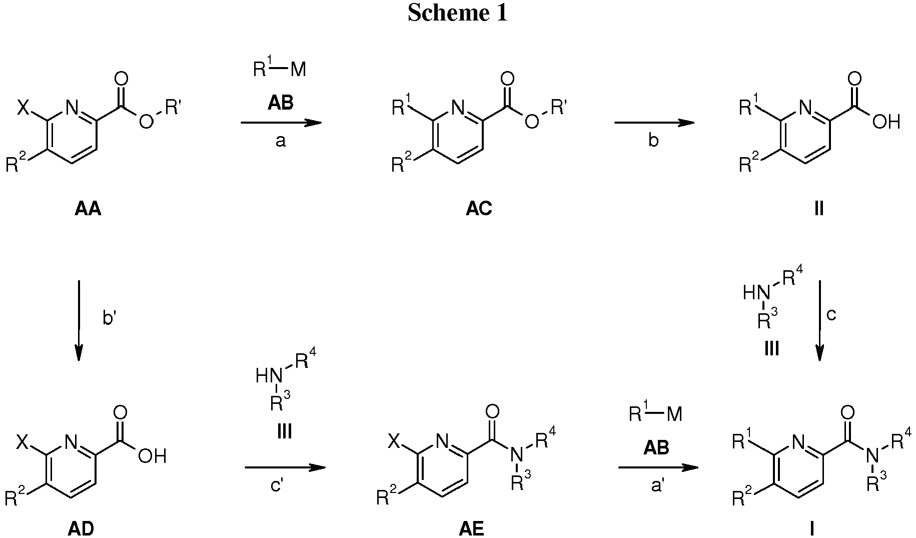

Compound **AC** can be prepared from **AA** by coupling a suitably substituted aryl metal species of formula **AB** (M is e.g. a trifluoroborate [BF₃]⁻K⁺, a boronic acid B(OH)₂ or a boronic acid pinacol ester group) (step a), particularly an arylboronic acid or arylboronic acid ester group in the presence of a suitable catalyst, in particular a palladium catalyst and more particularly palladium(II)acetate / triphenylphosphine mixtures or palladium(II)chloride-dppf (1,1'-bis(diphenylphosphino)ferrocene) complexes and a base such as triethylamine, sodium carbonate or potassium phosphate in an inert solvent such as dimethylformamide, toluene, tetrahydrofuran, acetonitrile and dimethoxyethane.

The saponification of the ester of general formula **AC** (R' ≠ H) by methods well known to the ones skilled in the art - using e.g. aqueous LiOH, NaOH or KOH in tetrahydrofuran / ethanol or another suitable solvent at temperatures between 0°C and the reflux temperature of the solvent employed - leads to an acid of general formula **II** (step b).

Compound **I** can be prepared from **II** and the corresponding amine of formula **III** by suitable amide bond forming reactions (step c). These reactions are known in the art. For example coupling reagents like *N,N*'-carbonyl-diimidazole (CDI), *N,N'-*dicyclohexylcarbodiimide (DCC), 1-(3-dimethylaminopropyl)-3-ethylcarbodiimide hydrochloride (EDCI), 1-[bis(dimethylamino)-methylene]-*1H*-1,2,3-triazolo[4,5-b]pyridinium-3-oxide hexafluorophosphate (HATU), 1-hydroxy-1,2,3-benzotriazole (HOBT), O-benzotriazol-1-yl-*N,N,N',N'*-tetramethyluronium tetrafluoroborate (TBTU), and O-benzotriazole-*N,N,N',N*'-tetramethyl-uronium-hexafluoro-phosphate (HBTU) can be employed to affect such transformation. A convenient method is to use for example HBTU and a base, for example *N*-methylmorpholine in an inert solvent such as for example dimethylformamide at room temperature.

Alternatively esters of general formula **AA** (R' ≠ H) can be saponified by methods well known to the ones skilled in the art - using e.g. aqueous LiOH, NaOH or KOH in tetrahydrofuran / ethanol or another suitable solvent at temperatures between 0°C and the reflux temperature of the solvent employed - to give acids of general formula **AD** (step b').

Compounds **AE** can be prepared from **AD** and the corresponding amine of formula **III** by suitable amide bond forming reactions (step c'). These reactions are known in the art. For example coupling reagents like *N,N'*-carbonyl-diimidazole (CDI), *N,N'-*dicyclohexylcarbodiimide (DCC), 1-(3-dimethylaminopropyl)-3-ethylcarbodiimide hydrochloride (EDCI), 1-[bis(dimethylamino)-methylene]-*1H*-1,2,3-triazolo[4,5-b]pyridinium-3-oxide hexafluorophosphate (HATU), 1-hydroxy-1,2,3-benzotriazole (HOBT), O-benzotriazol-1-yl-*N,N,N',N'*-tetramethyluronium tetrafluoroborate (TBTU), and O-benzotriazole-*N,N,N',N'*-tetramethyl-uronium-hexafluoro-phosphate (HBTU) can be employed to affect such transformation. A convenient method is to use for example HBTU and a base, for example *N*-methylmorpholine in an inert solvent such as for example dimethylformamide at room temperature.

Compound **I** can be prepared from **AE** by coupling a suitably substituted aryl metal species of formula **AB** (step a'), particularly an arylboronic acid or arylboronic acid ester in the presence of a suitable catalyst, in particular a palladium catalyst and more particularly palladium(II)acetate / triphenylphosphine mixtures or palladium(II)chloride-dppf (1,1'-bis(diphenylphosphino)ferrocene) complexes and a base such as triethylamine, sodium carbonate or potassium phosphate in an inert solvent such as dimethylformamide, toluene, tetrahydrofuran, acetonitrile and dimethoxyethane.

Amines **III** are either commercially available, described in the literature, can be synthesized by a person skilled in the art or as described in the experimental part.

If one of the starting materials, compounds of formulae **AA**, **AB** or **III**, contains one or more functional groups which are not stable or are reactive under the reaction conditions of one or more reaction steps, appropriate protecting groups (P) (as described e.g. in T.W. Greene et al., Protective Groups in Organic Chemistry, John Wiley and Sons Inc. New York 1999, 3rd edition) can be introduced before the critical step applying methods well known in the art. Such protecting groups can be removed at a later stage of the synthesis using standard methods known in the art.

If one or more compounds of formulae **AA** to **AE**, **II** or **III** contain chiral centers, picolines of formula **I** can be obtained as mixtures of diastereomers or enantiomers, which can be separated by methods well known in the art, e.g. (chiral) HPLC or crystallization. Racemic compounds can e.g. be separated into their antipodes via diastereomeric salts by crystallization or by separation of the antipodes by specific chromatographic methods using either a chiral adsorbent or a chiral eluent.

Following the procedure according to scheme 2, compound **BA** (R' = H, methyl, ethyl, isopropyl, tert. butyl or another suitable protecting group described for example in T.W. Greene et al., Protective Groups in Organic Chemistry, John Wiley and Sons Inc. New York 1999, 3rd edition) can be used as starting material. **BA** is either commercially available, described in the literature or can be synthesized by a person skilled in the art.

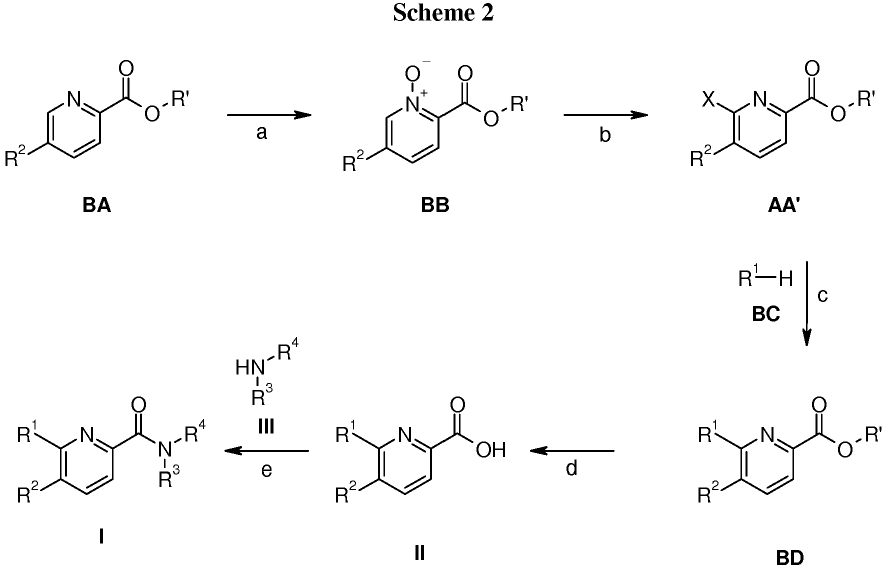

Compound **BB** can be prepared from **BA** by oxidation with a suitable oxidizing reagent under conditions known to a person skilled in the art (step a), e.g. by treatment with 3-chloro perbenzoic acid in dichloromethane at ambient temperature.

Conversion of compound **BB** to 6-chloro or 6-bromo-picoline **AA**' (X = Cl, Br) can be achieved e.g. by treatment with phosphoryl trichloride or tribromide either without an additional solvent or in a suitable solvent such as chloroform at temperatures between 20°C and the boiling point of the solvent, or by using other conditions known in the literature (step b).

6-Chloro- or bromo-picoline **AA'** (X = Cl, Br) can be transformed to compound **BD** by reaction with a suitably substituted primary or secondary alcohol **BC** in the presence of a base, for example sodium hydride, with or without an inert solvent, for example dimethylformamide, at temperatures ranging from room temperature to the reflux temperature of the solvent, particularly at room temperature (step c).

Compound **BD** can be further elaborated to compound **I** by: i) saponification (for compounds BD with R' ≠ H) as described in step b of scheme 1 (step d); ii) amide bond formation as described in step c of scheme 1 (step e).

Alternatively, compound AA' (R' = methyl, ethyl, isopropyl, tert. butyl or another suitable protecting group described for example in T.W. Greene et al., Protective Groups in Organic Chemistry, John Wiley and Sons Inc. New York 1999, 3rd edition) can be: i) converted into its acid congener AA' (R' = H) as described in step b of scheme 1; ii) transformed into the corresponding amide by treatment with amine **III** as described in step c of scheme 1; and iii) reacted with alcohol **BC** as described in step c to arrive at compound **I**.

If one of the starting materials, compounds of formulae **BA**, **BC** or **III**, contains one or more functional groups which are not stable or are reactive under the reaction conditions of one or more reaction steps, appropriate protecting groups (P) (as described e.g. in T.W. Greene et al., Protective Groups in Organic Chemistry, John Wiley and Sons Inc. New York 1999, 3rd edition) can be introduced before the critical step applying methods well known in the art. Such protecting groups can be removed at a later stage of the synthesis using standard methods known in the art.

If one or more compounds of formulae **BA** to **BD**, **AA'**, **II** or **III** contain chiral centers, picolines of formula **I** can be obtained as mixtures of diastereomers or enantiomers, which can be separated by methods well known in the art, e.g. (chiral) HPLC or crystallization. Racemic compounds can e.g. be separated into their antipodes via diastereomeric salts by crystallization or by separation of the antipodes by specific chromatographic methods using either a chiral adsorbent or a chiral eluent.

Following the procedure according to scheme 3, compound **CA** (R' = H, methyl, ethyl, isopropyl, tert. butyl or another suitable protecting group described for example in T.W. Greene et al., Protective Groups in Organic Chemistry, John Wiley and Sons Inc. New York 1999, 3rd edition) can be used as starting material. **CA** is either commercially available (e.g. for R' = methyl: 5-bromo-6-chloro-pyridine-2-carboxylic acid methyl ester CAN 1214353-79-3), described in the literature or can be synthesized by a person skilled in the art.

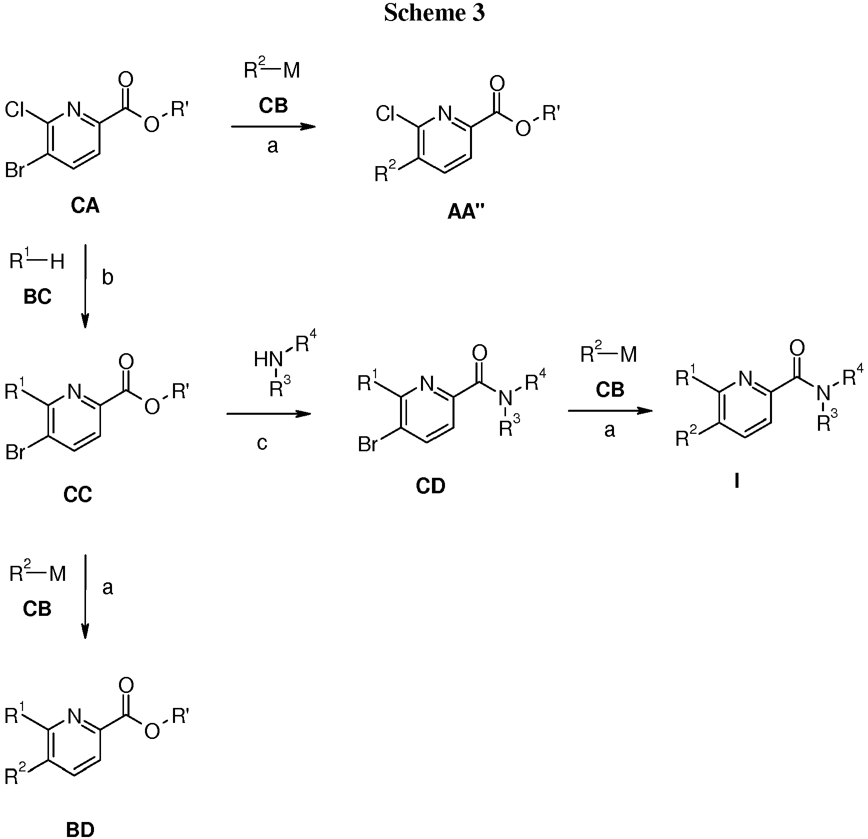

Compound **AA**" can be prepared from **CA** by coupling a suitably substituted aryl metal species of formula **CB** (M is e.g. a trifluoroborate [BF₃]⁻K⁺, a boronic acid B(OH)₂ or a boronic acid pinacol ester group) (step a), e.g. an organotrifluoroborate potassium salt in the presence of a palladium catalyst such as palladium(II)acetate / butyl-1-adamantylphosphine and a base such as cesium carbonate in an inert solvent such as toluene at temperatures between 50°C and the boiling temperature of the solvent, or an arylboronic acid or arylboronic acid ester in the presence of a suitable catalyst, in particular a palladium catalyst and more particularly palladium(II)acetate / triphenylphosphine mixtures or palladium(II)chloride-dppf (1,1'-bis(diphenylphosphino)ferrocene) complexes and a base such as triethylamine, sodium carbonate or potassium phosphate in an inert solvent such as dimethylformamide, toluene, tetrahydrofuran, acetonitrile or dimethoxyethane. Optionally, compound **CB** can also be an amine which is coupled to **CA** by methods well known to a person skilled in the art, e.g. using a palladium catalyst such as tris(dibenzylideneacetone)dipalladium / dimethylbisdiphenyl-phosphinoxanthene and a base such as cesium carbonate in a solvent such as 1,4-dioxane, preferentially at the boiling point of the solvent.

Compound **AA'** can be further elaborated to compound **I** by: i) reaction with compound **BC** to form compound **BD** as described in step c of scheme 2; ii) saponification as described in step b of scheme 1; and iii) amide bond formation as described in step c of scheme 1.

Furthermore, compound **CA** can be converted into compound **CC** by treatment with compound **BC** as described in step c of scheme 2 (step b).

Subsequent transformation of compound **CC** into compound **BD** can be achieved as discussed for the conversion of **CA** into **AA"** (step a).

Compound **BD** can be further elaborated to compound **I** by: i) saponification as described in step b of scheme 1; ii) amide bond formation as described in step c of scheme 1.

Alternatively, compound **CC** (R' = methyl, ethyl, isopropyl, tert. butyl or another suitable protecting group described for example in T.W. Greene et al., Protective Groups in Organic Chemistry, John Wiley and Sons Inc. New York 1999, 3rd edition) can be: i) converted into its acid congener **CC** (R' = H) as described in step b of scheme 1; ii) transformed into the corresponding amide **CD** by treatment with amine **III** as described in step c of scheme 1; and iii) reacted with **CB** as described in step a to arrive at compound **I**.

Furthermore, compound **I** can also be synthesized applying the following reaction sequence: i) saponification of compound **CA** (R' = methyl, ethyl, isopropyl, tert. butyl or another suitable protecting group described for example in T.W. Greene et al., Protective Groups in Organic Chemistry, John Wiley and Sons Inc. New York 1999, 3rd edition) to its acid congener **CC** (R' = H) as described in step b of scheme 1; ii) conversion to the corresponding amide by treatment with amine **III** as described in step c of scheme 1; iii) reaction with compound **CB** as described in step a; and iv) reaction with compound **BC** as described in step c. Optionally step iii) and step iv) can be interchanged.

If one of the starting materials, compounds of formulae **CA**, **CB** or **BC** contains one or more functional groups which are not stable or are reactive under the reaction conditions of one or more reaction steps, appropriate protecting groups (P) (as described e.g. in T.W. Greene et al., Protective Groups in Organic Chemistry, John Wiley and Sons Inc. New York 1999, 3rd edition) can be introduced before the critical step applying methods well known in the art. Such protecting groups can be removed at a later stage of the synthesis using standard methods known in the art.

If one or more compounds of formulae **CA**, **CB** or **BC** contain chiral centers, picolines of formula **AA"** and **BD** can be obtained as mixtures of diastereomers or enantiomers, which can be separated by methods well known in the art, e.g. (chiral) HPLC or crystallization. Racemic compounds can e.g. be separated into their antipodes via diastereomeric salts by crystallization or by separation of the antipodes by specific chromatographic methods using either a chiral adsorbent or a chiral eluent.

Following the procedure according to scheme 4, compound **DA** (X = Cl, Br, I or trifluoromethanesulfonate; R' = H, methyl, ethyl, isopropyl, tert. butyl or another suitable protecting group described for example in T.W. Greene et al., Protective Groups in Organic Chemistry, John Wiley and Sons Inc. New York 1999, 3rd edition) can be used as starting material. **DA** is either commercially available, described in the literature or can be synthesized by a person skilled in the art.

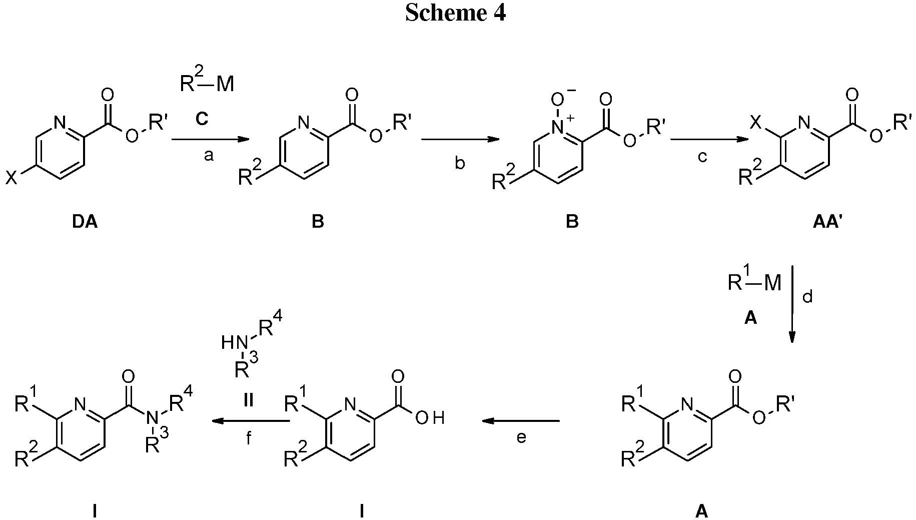

Compound **BA** can be prepared from **DA** by coupling a suitably substituted aryl metal species of formula **CB** (M is e.g. a trifluoroborate [BF₃]⁻K⁺, a boronic acid B(OH)₂ or a boronic acid pinacol ester group) (step a), e.g. an organotrifluoroborate potassium salt in the presence of a palladium catalyst such as palladium(II)acetate / butyl-1-adamantylphosphine and a base such as cesium carbonate in an inert solvent such as toluene at temperatures between 50°C and the boiling temperature of the solvent, or an arylboronic acid or arylboronic acid ester in the presence of a suitable catalyst, in particular a palladium catalyst and more particularly palladium(II)acetate / triphenylphosphine mixtures or palladium(II)chloride-dppf (1,1'-bis(diphenylphosphino)ferrocene) complexes and a base such as triethylamine, sodium carbonate or potassium phosphate in an inert solvent such as dimethylformamide, toluene, tetrahydrofuran, acetonitrile and dimethoxyethane. Optionally, compound **CB** can also be an amine which is coupled to **DA** by methods well known to a person skilled in the art, e.g. using a palladium catalyst such as tris(dibenzylideneacetone)dipalladium / dimethylbisdiphenyl-phosphinoxanthene and a base such as cesium carbonate in a solvent such as 1,4-dioxane preferentially at the boiling point of the solvent. Additionally, compounds AA' can be converted to thioethers AC through reaction with a thiole AB (M is H) applying methods well known to a person skilled in the art, e.g. using the sodium salt of a thiol in a solvent such as sulfolane at temperatures between 0 °C and the boiling point of the solvent.

Compound **BB** can be prepared from **BA** by oxidation with a suitable oxidizing reagent as described in step a of scheme 2 (step b).

Conversion of compound **BB** to 6-chloro- or 6-bromo-picoline **AA**' (X = Cl, Br) can be achieved as described in step b of scheme 2 (step c).

Compound **AC** can be prepared from **AA'** by coupling a suitably substituted aryl metal species of formula **AB** (M is e.g. a trifluoroborate [BF₃]⁻K⁺, a boronic acid B(OH)₂ or a boronic acid pinacol ester group) (step d), particularly an arylboronic acid or arylboronic acid ester in the presence of a suitable catalyst, in particular a palladium catalyst and more particularly palladium(II)acetate / triphenylphosphine mixtures or palladium(II)chloride-dppf (1,1'-bis(diphenylphosphino)ferrocene) complexes and a base such as triethylamine, sodium carbonate or potassium phosphate in an inert solvent such as dimethylformamide, toluene, tetrahydrofuran, acetonitrile and dimethoxyethane. Compound **AC** can be further elaborated to compound **I** by: i) saponification as described in step b of scheme 1 (step e); ii) amide bond formation as described in step c of scheme 1 (step f).

If one of the starting materials, compounds of formulae **DA**, **CB**, **AB** or **III**, contains one or more functional groups which are not stable or are reactive under the reaction conditions of one or more reaction steps, appropriate protecting groups (P) (as described e.g. in T.W. Greene et al., Protective Groups in Organic Chemistry, John Wiley and Sons Inc. New York 1999, 3rd edition) can be introduced before the critical step applying methods well known in the art. Such protecting groups can be removed at a later stage of the synthesis using standard methods known in the art.

If one or more compounds of formulae **DA**, **CB**, **BA**, **BB**, **AA'**, **AB**, **AC**, **II** or **III** contain chiral centers, picolines of formula **I** can be obtained as mixtures of diastereomers or enantiomers, which can be separated by methods well known in the art, e.g. (chiral) HPLC or crystallization. Racemic compounds can e.g. be separated into their antipodes via diastereomeric salts by crystallization or by separation of the antipodes by specific chromatographic methods using either a chiral adsorbent or a chiral eluent.

The invention also relates to a process for the preparation of a compound of formula (I) comprising the reaction of a compound of formula (A) 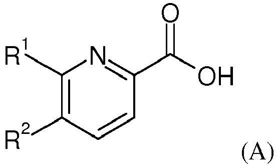 in the presence of NHR³R⁴, an amide bond forming coupling agent and a base, wherein R¹ to R⁴ are as defined above.

Examples of amide bond forming coupling agents are *N,N'*-carbonyl-diimidazole (CDI), *N,N'*-dicyclohexylcarbodiimide (DCC), 1-(3-dimethylaminopropyl)-3-ethylcarbodiimide hydrochloride (EDCI), 1-[bis(dimethylamino)-methylene]-*1H*-1,2,3-triazolo[4,5-b]pyridinium-3-oxide hexafluorophosphate (HATU), 1-hydroxy-1,2,3-benzotriazole (HOBT), O-benzotriazol-1-yl-*N,N,N',N'*-tetramethyluronium tetrafluoroborate (TBTU) and O-benzotriazole-*N,N,N',N'*-tetramethyl-uronium-hexafluoro-phosphate (HBTU).

Examples of suitable bases are tertiary amine bases like triethylamine, *N-*methylmorpholine, *N,N*-diisopropylethylamine or 4-(dimethylamino)-pyridine.

The reaction temperature is for example room temperature.

A convenient method is to use for example TBTU and a base, for example *N*-ethyl-*N*-isopropylpropan-2-amine in an inert solvent such as for example dimethylformamide at room temperature.

Another embodiment of the invention provides a pharmaceutical composition or medicament containing a compound of the invention and a therapeutically inert carrier, diluent or excipient, as well as a method of using the compounds of the invention to prepare such composition and medicament. In one example, the compound of formula (I) may be formulated by mixing at ambient temperature at the appropriate pH, and at the desired degree of purity, with physiologically acceptable carriers, i.e., carriers that are nontoxic to recipients at the dosages and concentrations employed into a galenical administration form. The pH of the formulation depends mainly on the particular use and the concentration of compound, but preferably ranges anywhere from about 3 to about 8. In one example, a compound of formula (I) is formulated in an acetate buffer, at pH 5. In another embodiment, the compound of formula (I) is sterile. The compound may be stored, for example, as a solid or amorphous composition, as a lyophilized formulation or as an aqueous solution.

Compositions are formulated, dosed, and administered in a fashion consistent with good medical practice. Factors for consideration in this context include the particular disorder being treated, the particular mammal being treated, the clinical condition of the individual patient, the cause of the disorder, the site of delivery of the agent, the method of administration, the scheduling of administration, and other factors known to medical practitioners.

The compounds of the invention may be administered by any suitable means, including oral, topical (including buccal and sublingual), rectal, vaginal, transdermal, parenteral, subcutaneous, intraperitoneal, intrapulmonary, intradermal, intrathecal and epidural and intranasal, and, if desired for local treatment, intralesional administration. Parenteral infusions include intramuscular, intravenous, intraarterial, intraperitoneal, or subcutaneous administration.

The compounds of the present invention may be administered in any convenient administrative form, e.g., tablets, powders, capsules, solutions, dispersions, suspensions, syrups, sprays, suppositories, gels, emulsions, patches, etc. Such compositions may contain components conventional in pharmaceutical preparations, e.g., diluents, carriers, pH modifiers, sweeteners, bulking agents, and further active agents.

A typical formulation is prepared by mixing a compound of the present invention and a carrier or excipient. Suitable carriers and excipients are well known to those skilled in the art and are described in detail in, e.g., Ansel, Howard C., et al., Ansel's Pharmaceutical Dosage Forms and Drug Delivery Systems. Philadelphia: Lippincott, Williams & Wilkins, 2004; Gennaro, Alfonso R., et al. Remington: The Science and Practice of Pharmacy. Philadelphia: Lippincott, Williams & Wilkins, 2000; and Rowe, Raymond C. Handbook of Pharmaceutical Excipients. Chicago, Pharmaceutical Press, 2005. The formulations may also include one or more buffers, stabilizing agents, surfactants, wetting agents, lubricating agents, emulsifiers, suspending agents, preservatives, antioxidants, opaquing agents, glidants, processing aids, colorants, sweeteners, perfuming agents, flavoring agents, diluents and other known additives to provide an elegant presentation of the drug (i.e., a compound of the present invention or preservatives, antioxidants, opaquing agents, glidants, processing aids, colorants, sweeteners, perfuming agents, flavoring agents, diluents and other known additives to provide an elegant presentation of the drug (i.e., a compound of the present invention or pharmaceutical composition thereof) or aid in the manufacturing of the pharmaceutical product (i.e., medicament).

The invention thus also relates to:
A compound of formula (I) for use as therapeutically active substance;
A pharmaceutical composition comprising a compound of formula (I) and a therapeutically inert carrier; and
A compound of formula (I) for use in the treatment or prophylaxis of pain, neuropathic pain, asthma, osteoporosis, inflammation, psychiatric diseases, psychosis, oncology, encephalitis, malaria, allergy, immunological disorders, arthritis, gastrointestinal disorders, psychiatric disorders rheumatoid arthritis, psychosis or allergy.

The invention will now be illustrated with the following examples which have no limiting character.

### Examples

### Abbreviations

BINAP = 2,2'-Bis(diphenylphosphino)-1,1'-binaphthyl; bp = boiling point; DIEA = N-ethyl-N-isopropylpropan-2-amine; DMF = dimethylformamide; DMSO = dimethylsulfoxide; dppf = 1,1'-bis(diphenylphosphino)ferrocene; EI = electron impact; EtOAc = ethyl acetate; HATU = 2-(3H-[1,2,3]triazolo[4,5-b]pyridin-3-yl)-1,1,3,3-tetramethylisouronium hexafluorophosphate(V); HBTU = O-benzotriazole-N,N,N',N'-tetramethyl-uronium-hexafluoro-phosphate; HPLC = LC = high performance liquid chromatography; iPrOAc = isopropyl acetate; ISP = ion spray, corresponds to ESI (electrospray); MS = mass spectrometry; NMM = *N*-methyl morpholine; NMR data are reported in parts per million (δ) relative to internal tetramethylsilane and are referenced to the deuterium lock signal from the sample solvent (d₆-DMSO unless otherwise stated); coupling constants (J) are in Hertz; *m*-CPBA = meta-chloroperoxybenzoic acid; mp = melting point; MTBE = 2-methoxy-2-methylpropane; Rt = retention time; TBTU = O-(benzotriazol-1-yl)-*N,N,N',N'*-tetramethyl-uronium-tetrafluoroborate; TEMPO = 2,2,6,6-tetra-methylpiperidine 1-oxyl radical; THF = tetrahydrofuran; tlc = thin layer chromatography.

### Example 1

### Methyl 2-[[5-(4-chlorophenyl)-6-methylsulfanylpyridine-2-carbonyl]amino]-2-methylpropanoate

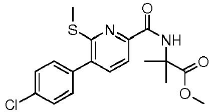

### a) Methyl 5-(4-chlorophenyl)-1-oxido-pyridin-1-ium-2-carboxylate

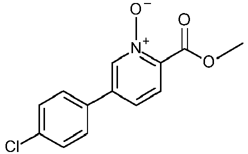

Methyl 5-(4-chlorophenyl)pyridine-2-carboxylate (CAN 86574-76-7, 4.5 g, 18.2 mmol) was dissolved in dichloromethane (50 mL) under an argon atmosphere to give a brown solution. 3-Chloroperoxybenzoic acid (4.92 g, 28.5 mmol) was added under stirring in five portions over 1 h. The reaction mixture was stirred under argon for 18 h. Additional 3-chloroperoxybenzoic acid (2.35 g, 13.6 mmol) was added and stirring was continued for 24 h. The reaction mixture was poured into 500 mL cold aqueous 10% Na₂SO₃ solution and extracted with CH₂Cl₂ (2 x 500 mL). The organic layers were washed with sat. aqueous NaHCO₃ solution (1 x 500 mL), and brine (1 x 400 mL). The organic layers were combined, dried over Na₂SO₄ and concentrated *in vacuo* to a volume of 30 mL. Heptane (50 mL) was added under stirring. A solid precipitated. Stirring was continued for 0.5 h, the solid was filtered off, washed with 30 mL heptane and dried under reduced pressure to obtain the title compound (3.45 g, 72%) as off- white solid, LC-MS (UV peak area/ESI) 91%, 264.0420[MH⁺].

### b) 6-Chloro-5-(4-chlorophenyl)pyridine-2-carboxylic acid methyl ester

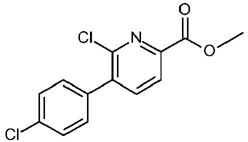

Phosphorus oxychloride (7.85 g, 4.69 mL, 51 mmol) was added to a solution of methyl 5-(4-chlorophenyl)-1-oxido-pyridin-1-ium-2-carboxylate (example 1a, 1.5 g, 5.69 mmol) in chloroform (4 mL). The reaction mixture was stirred at 80 °C for 18 h, poured into ice cold saturated aqueous K₂CO₃ solution (150 mL) and extracted with CH₂Cl₂ (2 x 200 mL). The combined organic layers were washed with brine (2 x 100 mL), dried over MgSO₄ and concentrated *in vacuo.* The crude material was purified by flash chromatography (silica gel, 70 g, 0% to 100% isopropyl acetate in heptane) and the resulting material triturated with 10 mL iPrOAc / heptane 9:1. The solid was filtered off and dried *in vacuo* to obtain the title compound (777 mg, 48%) as colorless solid, LC-MS (UV peak area/ESI) 91%, 282.008 [MH⁺].

### c) 5-(4-Chlorophenyl)-6-methylsulfanyl-pyridine-2-carboxylic acid

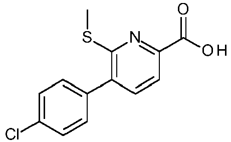

Sodium thiomethoxide (62.1 mg, 886 µmol) was added to a solution of 6-chloro-5-(4-chlorophenyl)pyridine-2-carboxylic acid methyl ester (example 1b, 50 mg, 177 µmol) in sulfolane (1.5 mL) under an argon atmosphere. The mixture was stirred for 24 h at ambient temperature, poured onto ice water / isopropyl acetate 1/1 and acidified with 1 N HCl. The organic layer was dried over Na₂SO₄, filtered off and concentrated *in vacuo* to obtain crude title compound (51 mg, quant.) as yellow solid which was used in the next reaction step without further purification.

### d) Methyl 2-[[5-(4-chlorophenyl)-6-methylsulfanylpyridine-2-carbonyl]amino]-2-methylpropanoate

A solution of 5-(4-chlorophenyl)-6-methylsulfanyl-pyridine-2-carboxylic acid (example 1c, 52 mg, 186 µmol), methyl 2-aminoisobutyrate (CAN 13257-67-5, 79 mg, 670 µmol), HATU (255 mg, 670 µmol) and DIEA (87 mg, 116 µL, 670 µmol) in DMF (1.5 mL) was stirred at ambient temperature for 3 days. The mixture was poured into isopropyl acetate and washed with water, sat. aqueous Na₂CO₃ and 1N HCl. The organic layer was dried over Na₂SO₄, filtered off and concentrated *in vacuo* to obtain a yellow oil which was purified by flash chromatography (silica gel, 50 g, 0% to 100% isopropyl acetate in heptane) to give the title compound (64 mg, 92%) as colorless waxy solid, MS (ISP): 379.2 [MH⁺].

### Example 2

### Methyl 2-[[5-(4-chlorophenyl)-6-(cyclopropylmethoxy)pyridine-2-carbonyl]amino]-2-methylpropanoate

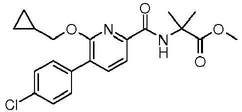

### a) 5-(4-Chlorophenyl)-6-(cyclopropylmethoxy)pyridine-2-carboxylic acid

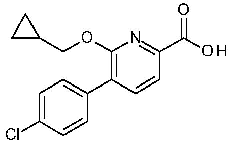

Under a nitrogen atomsphere, a solution of 5-bromo-6-(cyclopropylmethoxy)pyridine-2-carboxylic acid (CAN 1415898-37-1, 100 mg, 0.37 mmol), 4-chlorophenylboronic acid (CAN 1679-18-1, 57 mg, 0.40 mmol), Pd(dppf)Cl₂xCH₂Cl₂ (14 mg, 0.02 mmol), Na₂CO₃ (2 N, 291 mg, 3 mmol) in DMF (5 mL) was reacted overnight at 100 °C. The reaction mixture was poured into water and extracted with ethyl acetate (20 mL). The aqueous layer was adjusted to pH 2 by concentrated HCl, extracted with ethyl acetate (3 x 20 mL), washed with brine (6 x 20 mL), dried over Na₂SO₄ and concentrated under reduced pressure. The crude product was purified by chromatography over silica gel using petroleum ether/ethyl acetate = 4/1 to give the title compound (0.05 g, 49%) as a yellow solid.

### b) Methyl 2-[[5-(4-chlorophenyl)-6-(cyclopropylmethoxy)pyridine-2-carbonyl]amino]-2-methylpropanoate

To a solution of 5-(4-chlorophenyl)-6-(cyclopropylmethoxy)pyridine-2-carboxylic acid (example 2a, 100 mg, 0.325 mmol) in DMF (5 mL) was added NMM (131 mg, 1.3 mmol) and HBTU (247 mg, 0.65 mmol) at ambient temperature. The mixture was stirred for 1 hour at ambient temperature. Methyl 2-aminoisobutyrate (CAN 13257-67-5, 41 mg, 0.352 mmol), was added to the mixture. The solution was stirred at ambient temperature overnight, diluted with water (15 mL), extracted with EtOAc (3 x 15 mL). The combined organic layer was washed with water (2 x 10 mL) and brine (10 mL) and evaporated to dryness. The residue was purified by silica gel chromatography eluting with petroleumn ether/ethyl acetate = 8:1 to obtain the title compound (0.4 g, 30%) as white solid, LC-MS: 403.1 [MH⁺]. ¹H NMR (300 MHz, *CDCl₃*): *δ* 8.47 (bs, 1H), 7.81 (d, 1H, J = 7.5 Hz), 7.74 (d, 1H, J = 7.5 Hz), 7.56 (dd, 2H, J₁ = 6.6 Hz, J₂ =1.8 Hz), 7.41 (dd, 2H, J₁ = 6.9 Hz, J₂ = 2.1 Hz), 4.30 (d, 2H, J = 5.1 Hz), 3.80 (s, 3H), 1.72 (s, 6H), 1.40 - 1.20 (m, 1H), 0.64 - 0.60 (m, 2H), 0.42 - 0.38 (m, 2H).

### Example 3

### 5-(4-Chlorophenyl)-6-(cyclopropylmethoxy)-N-[2-(1,3-oxazol-2-yl)propan-2-yl]pyridine-2-carboxamide

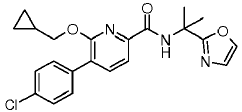

In analogy to the procedure described in example 2b, 5-(4-chlorophenyl)-6-(cyclopropylmethoxy)pyridine-2-carboxylic acid (example 2a) was condensed with 2-oxazol-2-ylpropan-2-amine (CAN 1211519-76-4) to obtain the title compound (83 mg, 32%) as colorless oil, LC-MS: 411.9 [MH⁺]. ¹H NMR (300 MHz, *CDCl₃*): *δ* 8.74 (s, 1H), 7.80 - 7.71 (m, 2H), 7.57 - 7.53 (m, 3H), 7.42 - 7.39 (m, 2H), 7.10 (s, 1H), 4.31 (d, 2H, J = 6.9 Hz), 1.91 (s, 6H), 1.35 - 1.25 (m, 1H), 0.64 - 0.59 (m, 2H), 0.42 - 0.38 (m, 2H).

### Example 4

### 5-(4-Chlorophenyl)-6-(cyclopropylmethoxy)-N-[2-(5-methyl-1,2,4-oxadiazol-3-yl)propan-2-yl]pyridine-2-carboxamide

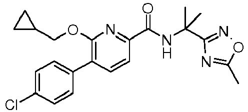

In analogy to the procedure described in example 2b, 5-(4-chlorophenyl)-6-(cyclopropylmethoxy)pyridine-2-carboxylic acid (example 2a) was condensed with 2-(5-methyl-1,2,4-oxadiazol-3-yl)propan-2-amine (CAN 1153831-97-0) to obtain the title compound (88 mg, 48%) as white solid, LC-MS: 427.1 [MH⁺]. ¹H NMR (300 MHz, *CDCl₃*): *δ* 8.58 (bs, 1H), 7.80 (d, 1H, J = 7.5 Hz), 7.73 (d, 1H, J = 7.5 Hz), 7.56 (d, 2H, J = 8.4 Hz), 7.41 (dd, 2H, J₁ = 6.6 Hz, J₂ = 1.8 Hz), 4.30 (d, 2H, J = 7.2 Hz), 2.60 (s, 3H), 1.89 (s, 6H), 1.40 - 1.20 (m, 1H), 0.64 - 0.62 (m, 2H), 0.40 - 0.37 (m, 2H).

### Example 5

### 6-(Cyclopropylmethoxy)-N-[2-(1,3-oxazol-2-yl)propan-2-yl]-5-phenylpyridine-2-carboxamide

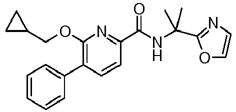

### a) 6-(Cyclopropylmethoxy)-5-phenyl-pyridine-2-carboxylic acid

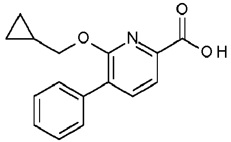

In analogy to the procedure described in example 2a, 5-bromo-6-(cyclopropylmethoxy)pyridine-2-carboxylic acid (CAN 1415898-37-1, 2 g, 7 mmol) was reacted with phenylboronic acid (CAN 98-80-6, 1.07 g, 9 mmol) to give the title compound (1.3 g, 66%) as white solid, LC-MS: 270.1 [MH⁺].

### b) 6-(Cyclopropylmethoxy)-N-[2-(1,3-oxazol-2-yl)propan-2-yl]-5-phenylpyridine-2-carboxamide

In analogy to the procedure described in example 2b, 6-(cyclopropylmethoxy)-5-phenylpyridine-2-carboxylic acid (example 5a) was condensed with 2-oxazol-2-ylpropan-2-amine (CAN 1211519-76-4) to obtain the title compound (41 mg, 29%) as white solid, LC-MS: 378.2 [MH⁺]. ¹H NMR (300 MHz, *CDCl₃*): *δ* 8.75 (s, 1H), 7.81 - 7.74 (m, 2H), 7.64 - 7.61 (m, 3H), 7.46 - 7.34 (m, 4H), 7.11 (s, 1H), 4.32 (d, 2H, J = 6.9 Hz), 1.91 (s, 6H), 1.43 - 1.37 (m, 1H), 0.65 - 0.59 (m, 2H), 0.43 - 0.39 (m, 2H).

### Example 6

### 6-(Cyclopropylmethoxy)-N-[3-(methylcarbamoyl)pentan-3-yl]-5-phenylpyridine-2-carboxamide

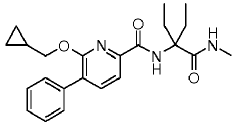

In analogy to the procedure described in example 2b, 6-(cyclopropylmethoxy)-5-phenylpyridine-2-carboxylic acid (example 5a) was condensed with 2-amino-2-ethyl-*N*-methylbutanamide (CAN 1415898-90-6) to obtain the title compound (29 mg, 20%) as white solid, LC-MS: 396.1 [MH⁺]. ¹H NMR (300 MHz, *CDCl₃*): 8.98 (bs, 1H), 7.81 (dd, 2H, J₁ = 12.0 Hz, J₂ = 7.5 Hz), 7.65 (dd, 2H, J₁ = 8.4 Hz, J₂ = 1.2 Hz), 7.49 - 7.37 (m, 3H), 6.21 (bs, 1H), 4.38 (d, 2H, J = 6.9 Hz), 2.93 (d, 3H, J = 4.5 Hz), 2.67 - 2.57 (m, 2H), 1.82 - 1.72 (m, 2H), 1.40 - 1.25 (m, 1H), 0.87 (t, 6H, J = 7.5 Hz), 0.68 - 0.62 (m, 2H), 0.42 - 0.38 (m, 2H).

### Example 7

### 6-(3-Chlorophenyl)-5-(cyclopropylmethoxy)-N-[3-(methylcarbamoyl)pentan-3-yl]pyridine-2-carboxamide

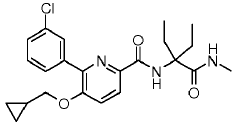

### a) 5-(Cyclopropylmethoxy)-1-oxido-pyridin-1-ium-2-carboxylic acid

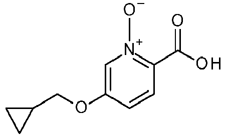

30% H₂O₂ (15 mL) was added to a solution of 5-(cyclopropylmethoxy)pyridine-2-carboxylic acid (CAN 1266787-40-9, 0.44 g, 2.28 mmol) in acetic acid (20 mL). The mixture was stirred overnight at 60 °C. The solvent was removed under reduced pressure to give the crude title compound (0.2 g, 42%), which was used in the next reaction step without further purification, LC-MS: 210.1 [MH⁺]. ¹H NMR (300 MHz, *CD₃OD*): *δ* 8.24 - 8.13 (m, 2H), 7.37 - 7.11 (m, 1H), 3.97 - 3.90, (m, 2H), 1.21 - 1.86 (m, 1H), 0.61 - 0.55 (m, 2H), 0.34 - 0.29 (m, 2H).

### b) 6-Bromo-5-(cyclopropylmethoxy)pyridine-2-carboxylic acid

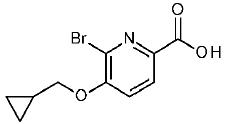

5-(Cyclopropylmethoxy)-1-oxido-pyridin-1-ium-2-carboxylic acid (example 7a, (3.0 g, 14.3 mmol) was added to a solution of POBr₃ (30 g) in dichloromethane (10 mL). The mixture was stirred overnight at 40 °C. Ice water was added, the mixture was extracted with dichloromethane (3 x 100 mL) and the organic layers were combined. The solvent was removed, 1N NaOH solution was added and the mixture was washed with dichloromethane (2 x 40 mL). The aqueous layer was acidified with 1N HCl, extracted with dichloromethane (3 x 100 mL), dried over Na₂SO₄ and concentrated to give the crude title compound (1.0 g, 32%). ¹H NMR (300 MHz, *CDCl₃*): *δ* 8.07 - 8.04 (m, 1H), 7.15 - 7.13 (m, 1H), 3.99 - 3.96, (m, 2H), 1.37 - 1.32 (m, 1H), 0.74 - 0.68 (m, 2H), 0.47 - 0.42 (m, 2H).

### c) 6-(3-Chlorophenyl)-5-(cyclopropylmethoxy)pyridine-2-carboxylic acid

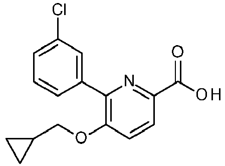

In analogy to the procedure described in example 2a, 6-bromo-5-(cyclopropylmethoxy)pyridine-2-carboxylic acid (example 7b, 0.3 g, 1.1 mmol) was reacted with 3-chlorophenylboronic acid (CAN 63503-60-6, 0.21 g, 1.3 mmol) to give the title compound (60 mg, 18%), LC-MS: 304.0 [MH⁺].

### d) 6-(3-Chlorophenyl)-5-(cyclopropylmethoxy)-N-[3-(methylcarbamoyl)pentan-3-yl]pyridine-2-carboxamide

In analogy to the procedure described in example 2b, 6-(3-chlorophenyl)-5-(cyclopropylmethoxy)pyridine-2-carboxylic acid (example 7d) was condensed with 2-amino-2-ethyl-N-methyl-butanamide (1415898-90-6) to obtain the title compound (5 mg, 9%) as white solid, LC-MS: 430.2 [MH⁺]. ¹H NMR (300 MHz, *CD₃OD*): *δ* 9.45 (bs, 1H), 8.21 - 8.07 (m, 2H), 8.01 (d, 1H, J = 8.7 Hz), 7.60 (d, 1H, J = 8.7 Hz), 7.51 - 7.41 (m, 2H), 4.05 (d, 2H, J = 6.9 Hz), 2.82 (s, 3H), 2.70 - 2.50 (m, 2H), 1.90 - 1.75 (m, 2H), 1.40 - 1.20 (m, 2H), 0.78 (t, 6H, J = 7.5 Hz), 0.70 - 0.65 (m, 2H), 0.45 - 0.40 (m, 2H).

### Example 8

### 6-(Cyclopropylmethoxy)-5-(3-methoxyazetidin-1-yl)-N-[2-(5-methyl-1,2,4-oxadiazol-3-yl)propan-2-yl]pyridine-2-carboxamide

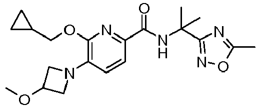

### a) 6-(Cyclopropylmethoxy)-5-(3-methoxyazetidin-1-yl)pyridine-2-carboxylic acid

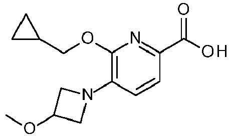

Under a nitrogen atmosphere, 3-methoxyazetidine (38 mg, 0.44 mmol), BINAP (23 mg, 0.037 mmol), Pd₂(dba)₃ (17 mg, 0.02 mmol) and Cs₂CO₃ (240 mg, 0.735 mmol) were added to a solution of 5-bromo-6-(cyclopropylmethoxy)pyridine-2-carboxylic acid (CAN 1415898-37-1, 100 mg, 0.37 mmol) in toluene (4 mL). The reaction mixture was stirred overnight at 110 °C and then concentrated under reduced pressure. The residue was dissolved in water and extracted with ethyl acetate (30 mL). The aqueous layer was adjusted to pH 2 by addition of 1N HCl. The resulting precipitate was collected by filtration and dried *in vacuo.* Chromatographical purification over silica gel using petroleum ether/ethyl acetate = 1/2 provided the title compound (35 mg, 34%) as a yellow solid, LC-MS: 265.2 [MH⁺].

### b) 6-(Cyclopropylmethoxy)-5-(3-methoxyazetidin-1-yl)-N-[2-(5-methyl-1,2,4-oxadiazol-3-yl)propan-2-yl]pyridine-2-carboxamide

In analogy to the procedure described in example 2b, 6-(cyclopropylmethoxy)-5-(3-methoxyazetidin-1-yl)pyridine-2-carboxylic acid (example 8a) was condensed with 2-(5-methyl-1,2,4-oxadiazol-3-yl)propan-2-amine (CAN 1153831-97-0) to obtain the title compound (47 mg, 33%) as colorless oil, LC-MS: 402.2 [MH⁺]. ¹H NMR (300 MHz, *CDCl₃*): *δ* 8.25 (bs, 1H), 7.59 (d, 1H, J = 8.1 Hz), 6.55 (d, 1H, J = 7.8 Hz), 4.35 - 4.15 (m, 5H), 3.90 - 3.80 (m, 2H), 3.33 (d, 3H, J = 1.2 Hz), 2.56 (s, 3H), 1.83 (s, 6H), 1.40 - 1.20 (m, 1H), 0.65 - 0.61 (m, 2H), 0.39 - 0.36 (m, 2H).

### Example 9

### 6-(3-Chlorophenyl)-5-(cyclopropylmethoxy)-N-[2-(5-methyl-1,2,4-oxadiazol-3-yl)propan-2-yl]pyridine-2-carboxamide

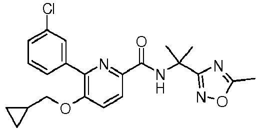

In analogy to the procedure described in example 2b, 6-(3-chlorophenyl)-5-(cyclopropylmethoxy)pyridine-2-carboxylic acid (example 7d) was condensed with 2-(5-methyl-1,2,4-oxadiazol-3-yl)propan-2-amine (CAN 1153831-97-0) to obtain the title compound (20 mg, 29%) as white solid, LC-MS: 427.2 [MH⁺]. ¹H NMR (300 MHz, *CD₃OD*): *δ* 8.11 (d, 1H, J = 1.8 Hz), 8.02 - 7.95 (m, 2H), 7.58 (d, 1H, J = 8.7 Hz), 7.50 - 7.40 (m, 2H), 4.04 (d, 2H, J = 6.9 Hz), 2.58 (s, 3H), 1.82 (s, 6H), 1.40 - 1.20 (m, 2H), 0.68 - 0.64 (m, 2H), 0.44 - 0.40 (m, 2H).

### Example 10

### N-[(2S)-1-amino-3-cyclopropyl-1-oxopropan-2-yl]-6-(cyclopropylmethoxy)-5-(3-methoxyazetidin-1-yl)pyridine-2-carboxamide

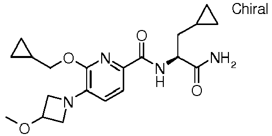

In analogy to the procedure described in example 2b, 6-(cyclopropylmethoxy)-5-(3-methoxyazetidin-1-yl)pyridine-2-carboxylic acid (example 8a) was condensed with (2S)-2-amino-3-cyclopropyl-propanamide (CAN 156077-93-9) to obtain the title compound (40 mg, 36%) as white solid, LC-MS: 389.1 [MH⁺]. ¹H NMR (300 MHz, *CDCl₃*): *δ* 8.05 (d, 1H, J = 7.8 Hz), 7.66 (d, 1H, J = 7.5 Hz), 6.57 (d, 1H, J = 7.8 Hz), 6.50 (bs, 1H), 5.45 (bs, 1H), 4.64 (dd, 1H, J₁ = 13.8 Hz, J₂ = 6.6 Hz), 4.35 - 4.25 (m, 3H), 4.14 (d, 2H, J = 7.2 Hz), 3.95 - 3.85 (m, 2H), 3.34 (s, 3H), 1.95 - 1.65 (m, 2H), 1.35 - 1.20 (m, 1H), 0.90 - 0.75 (m, 1H), 0.64 - 0.15 (m, 8H).

### Example 11

### 6-(Cyclopropylmethoxy)-5-(2-oxa-6-azaspiro[3.3]heptan-6-yl)-N-[2-(1,3-thiazol-2-yl)propan-2-yl]pyridine-2-carboxamide

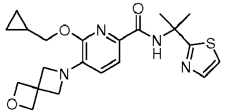

### a) 6-(Cyclopropylmethoxy)-5-(2-oxa-6-azaspiro[3.3]heptan-6-yl)pyridine-2-carboxylic acid methyl ester

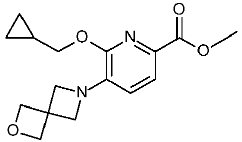

Under a nitrogen atmosphere a mixture of compound methyl 5-bromo-6-(cyclopropylmethoxy)pyridine-2-carboxylate (CAN 1415899-20-5, 0.51 g, 1.8 mmol), 2-oxa-6-azaspiro[3.3]heptane ethanedioate (CAN 1254966-66-9, 0.29 mg, 1.8 mmol), Pd₂(dba)₃ (33 mg, 0.035 mmol), BINAP (45 mg, 0.07 mmol) and Cs₂CO₃ (1.76 mg, 5.4 mmol) in toluene (50 mL) was stirred at 110 °C overnight. After concentration, the residue was partitioned between water (30 mL) and EtOAc (30 mL). The aqueous phase was extracted with EtOAc (2×20 mL). The combined organic phase was washed with brine (30 mL), dried over anhydrous Na₂SO₄, filtered and concentrated to give a residue which was purified by column chromatography eluting with petroleum ether - ethyl acetate (1:1) to give the target compound (0.4 g, 73%) as yellow solid, LC-MS: 305.1 [MH⁺].

### b) 6-(Cyclopropylmethoxy)-5-(2-oxa-6-azaspiro[3.3]heptan-6-yl)pyridine-2-carboxylic acid

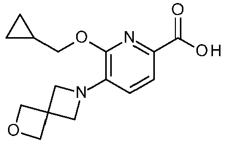

A mixture of 6-(cyclopropylmethoxy)-5-(2-oxa-6-azaspiro[3.3]heptan-6-yl)pyridine-2-carboxylic acid methyl ester (example 11a, 0.4 g, 1.3 mmol) and LiOHxH₂O (0.17 g, 3.9 mmol) in THF/H₂O (15 mL) was stirred at ambient temperature for 3 hours. After removal of the organic solvent, water (10 mL) was added to the residue and the mixture was extracted with EtOAc (3x15 mL). The combined organic phase was washed with brine (20 mL), dried over anhydrous Na₂SO₄, filtered and concentrated to give the target compound (0.38 g, 99%), LC-MS: 291.2 [MH⁺].

### c) 6-(Cyclopropylmethoxy)-5-(2-oxa-6-azaspiro[3.3]heptan-6-yl)-N-[2-(1,3-thiazol-2-yl)propan-2-yl]pyridine-2-carboxamide

In analogy to the procedure described in example 2b, 6-(cyclopropylmethoxy)-5-(2-oxa-6-azaspiro[3.3]heptan-6-yl)pyridine-2-carboxylic acid (example 11b) was condensed with 2-thiazol-2-ylpropan-2-amine (CAN 1082393-38-1) to obtain the title compound (20 mg, 15%) as white solid, LC-MS: 415.1 [MH⁺]. ¹H NMR (300 MHz, *CDCl₃*): *δ* 8.65 (bs, 1H), 7.68 (d, 1H, J = 3.0 Hz), 7.63 (d, 1H, J = 7.8 Hz), 7.25 (s, 1H), 6.56 (d, 1H, J = 7.8 Hz), 4.84 (s, 4H), 4.25 - 4.15 (m, 6H), 1.93 (s, 6H), 1.40 - 1.25 (m, 1H), 0.69 - 0.63 (m, 2H), 0.42 - 0.37 (m, 2H).

### Example 12

### N-[(2S)-1-amino-4-methyl-1-oxopentan-2-yl]-6-(cyclopropylmethoxy)-5-(2-oxa-6-azaspiro[3.3]heptan-6-yl)pyridine-2-carboxamide

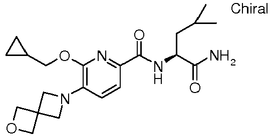

In analogy to the procedure described in example 2b, 6-(cyclopropylmethoxy)-5-(2-oxa-6-azaspiro[3.3]heptan-6-yl)pyridine-2-carboxylic acid (example 11b) was condensed with (2*S*)-2-amino-4-methyl-pentanamide (CAN 687-51-4) to obtain the title compound (76 mg, 71%) as light yellow solid, LC-MS: 403.2 [MH⁺]. ¹H NMR (300 MHz, *CDCl₃*): *δ* 7.72 (d, 1H, J = 8.1 Hz), 7.64 (d, 1H, J = 7.8 Hz), 6.56 (d, 1H, J = 7.8 Hz), 6.46 (bs, 1H), 5.41 (bs, 1H), 4.84 (s, 4H), 4.62 - 4.55 (m, 1H), 4.21 - 4.05 (m, 6H), 1.90 - 1.60 (m, 3H), 1.31 - 1.25 (m, 1H), 0.94 (t, 6H, J = 6.6 Hz), 0.68 - 0.62 (m, 2H), 0.40 - 0.35 (m, 2H).

### Example 13

### 6-(3-Chlorophenyl)-5-(cyclopropylmethoxy)-N-[2-(1,3-thiazol-2-yl)propan-2-yl]pyridine-2-carboxamide

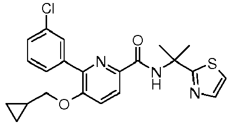

In analogy to the procedure described in example 2b, 6-(3-chlorophenyl)-5-(cyclopropylmethoxy)pyridine-2-carboxylic acid (example 7d) was condensed with 2-thiazol-2-ylpropan-2-amine (CAN 1082393-38-1) to obtain the title compound (20 mg, 28%) as white solid, LC-MS: 428.1 [MH⁺]. ¹H NMR (300 MHz, *CD₃OD*): *δ* 8.16 (d, 1H, J = 1.8 Hz), 8.06 - 8.03 (m, 1H), 7.98 (d, 1H, J = 8.4 Hz), 7.72 (d, 1H, J = 3.3 Hz), 7.60 (d, 1H, J = 8.7 Hz), 7.51 - 7.44 (m, 3H), 4.05 (d, 2H, J = 6.9 Hz), 1.93 (s, 6H), 1.40 - 1.20 (m, 1H), 0.70 - 0.60 (m, 2H), 0.45 - 0.40 (m, 2H).

### Example 14

### N-[(2S)-1-Amino-3-cyclopropyl-1-oxopropan-2-yl]-5,6-bis(cyclopropylmethoxy)pyridine-2-carboxamide

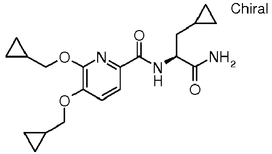

### a) 5,6-Bis(cyclopropylmethoxy)pyridine-2-carboxylic acid

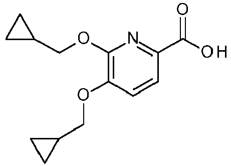

Sodium hydride (2.4 g, 0.1 mol) was added within 30 min to an ice-cold solution of cyclopropylmethanol (20 mL). Methyl 5-bromo-6-chloro-pyridine-2-carboxylate (CAN 1214353-79-3, 5 g, 0.02 mol) was added. The reaction mixture was heated to 100 °C for 2 hours. After cooling to ambient temperature, the reaction mixture was quenched by addition of water. The solvent was removed under reduced pressure, water was added and the pH was brought to 3 using 1N HCl. The resulting precipitate was collected by filtration, and the solution was extracted with ethyl acetate (3x30 mL). The combined organic layers were washed with brine (3x30mL), and dried over Na₂SO₄. After removal of the solvent by evaporation, the crude product was purified by silica gel chromatography eluting with petroleum ether/ethyl acetate = 1:1 to give the title compound (4.7 g, 86%) as a white solid, LC-MS: 264.2 [MH⁺].

### b) N-[(2S)-1-Amino-3-cyclopropyl-1-oxopropan-2-yl]-5,6-bis(cyclopropylmethoxy)pyridine-2-carboxamide

In analogy to the procedure described in example 2b, 5,6-bis(cyclopropylmethoxy)pyridine-2-carboxylic acid (example 14a) was condensed with (2*S*)-2-amino-3-cyclopropyl-propanamide (CAN 156077-93-9) to obtain the title compound (20 mg, 28%) as white solid, LC-MS: 374.2 [MH⁺]. ¹H NMR (300 MHz, *DMSO-d6*): *δ* 8.21 (d, 1H, J = 7.8 Hz), 7.60 - 7.25 (m, 2H), 7.34 (d, 1H, J = 8.1 Hz), 7.11 (bs, 1H), 4.44 (dd, 1H, J₁ = 13.2 Hz, J₂ = 7.5 Hz), 4.24 (d, 2H, J = 7.2 Hz), 3.89 (d, 2H, J = 6.9 Hz), 1.80 - 1.65 (m, 1H), 1.60 - 1.45 (m, 1H), 1.40 - 1.20 (m, 2H), 0.75 - 0.50 (m, 4H), 0.50 - 0.30 (m, 5H), 1.50 - 1.25 (m, 2H).

### Example 15

### N-[(2S)-1-Amino-4-methyl-1-oxopentan-2-yl]-6-(cyclopropylmethoxy)-5-(3-methoxyazetidin-1-yl)pyridine-2-carboxamide

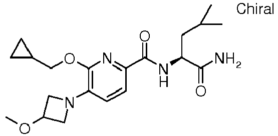

In analogy to the procedure described in example 2b, 6-(cyclopropylmethoxy)-5-(3-methoxyazetidin-1-yl)pyridine-2-carboxylic acid (example 8a) was condensed with (2*S*)-2-amino-4-methyl-pentanamide (CAN 687-51-4) to obtain the title compound (90 mg, 81%) as white solid, LC-MS: 391.2 [MH⁺]. ¹H NMR (300 MHz, *CDCl₃*): *δ* 7.75 (d, 1H, J = 8.4 Hz), 7.60 (d, 1H, J = 7.8 Hz), 7.11 (bs, 1H), 8.21 (d, 1H, J = 7.8 Hz), 6.76 (bs, 1H), 4.66 - 4.59 (m, 1H), 4.30 - 4.00 (m, 5H), 3.90 - 3.80 (m, 2H), 3.32 (s, 3H), 1.90 - 1.60 (m, 3H), 1.35 - 1.20 (m, 1H), 1.00 - 0.85 (m, 6H), 0.63 - 0.57 (m, 2H), 0.37 - 0.32 (m, 2H).

### Example 16

### N-[(2S)-1-Amino-4-methylsulfanyl-1-oxobutan-2-yl]-6-chloro-5-(cyclopropylmethoxy)pyridine-2-carboxamide

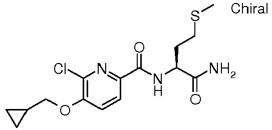

### a) 6-(Cyclopropylmethoxy)-5-(trifluoromethoxy)pyridine-2-carboxylic acid and 6-chloro-5-(cyclopropylmethoxy)pyridine-2-carboxylic acid

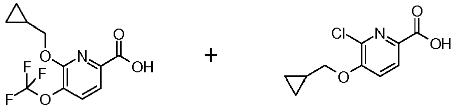

To a solution of 6-chloro-5-(trifluoromethoxy)-2-pyridinecarboxylic acid (CAN 1221171-90-9, 1. 0 g, 4.14 mmol) in DMSO (16 mL) was added powdered potassium hydroxide (929 mg, 16.6 mmol) and cyclopropylmethanol (335 µL, 4.14 mmol). After stirring for 16 hours at room temperature more cyclopropylmethanol (335 µL, 4.14 mmol) was added and the mixture was stirred for 4 hours at 50 °C. After cooling the mixture was added to 1 N sodium hydroxide solution/ice/water (50 mL) and washed with MTBE (100 mL). Organic phases were extracted with 1 N sodium hydroxide solution (20 mL). The water phases were combined, acidified with 2 N HCl (50 mL) and extracted with MTBE(2 x 150 mL). Organic phases were washed with icewater/brine (1:1, 2x 150 mL), combined, dried with Na2SO4, filtered and concentrated *in vacuo.* The residue was a 1:1 mixture of the title compounds as brown oil that solidified upon standing; LC-MS (UV peak area/ESI) 51.2%, 278.0628 [MH⁺] and 48.8%, 228.0425 [MH⁺].

### b) N-[(2S)-1-Amino-4-methylsulfanyl-1-oxobutan-2-yl]-6-chloro-5-(cyclopropylmethoxy)pyridine-2-carboxamide

A solution of the mixture of 6-(cyclopropylmethoxy)-5-(trifluoromethoxy)pyridine-2-carboxylic acid and 6-chloro-5-(cyclopropylmethoxy)pyridine-2-carboxylic acid (Example 16 a, 100 mg), (2S)-2-amino-4-(methylthio)-butanamide hydrochloride (1:1) (CAN 16120-92-6, 73.3 mg, 0.397 mmol), DIEA (309 µL, 1.8 mmol) and TBTU (127 mg, 0.397 mmol) in DMF (4 mL) was stirred for 20 h at room temperature. The crude reaction mixture was concentrated in vacuo and purified by flash chromatography (silica gel, 0% to 100% ethyl acetate in heptane) to give the title compound (32 mg, 25%) as light-yellow solid; LC-MS (UV peak area/ESI) 96%, 358.0988 [MH+].

### Example 17

### 6-Chloro-5-(cyclopropylmethoxy)-N-[3-(methylcarbamoyl)pentan-3-yl]pyridine-2-carboxamide

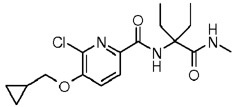

### a) 6-(Cyclopropylmethoxy)-5-(trifluoromethoxy)pyridine-2-carboxylic acid and 6-chloro-5-(cyclopropylmethoxy)pyridine-2-carboxylic acid

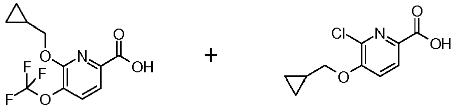

To a solution of 6-chloro-5-(trifluoromethoxy)-2-pyridinecarboxylic acid (CAN 1221171-90-9, 1. 0 g, 4.14 mmol) in DMSO (16 mL) was added powdered potassium hydroxide (929 mg, 16.6 mmol) and cyclopropylmethanol (335 µL, 4.14 mmol). After stirring for 16 hours at room temperature more cyclopropylmethanol (335 µL, 4.14 mmol) was added and the mixture was stirred for 4 hours at 50 °C. After cooling the mixture was added to 1 N sodium hydroxide solution/ice/water (50 mL) and washed with MTBE (100 mL). Organic phases were extracted with 1 N sodium hydroxide solution (20 mL). The water phases were combined, acidified with 2 N HCl (50 mL) and extracted with MTBE(2 x 150 mL). Organic phases were washed with icewater/brine (1:1, 2x 150 mL), combined, dried with Na₂SO₄, filtered and concentrated *in vacuo.* The residue was a 1:1 mixture of the title compounds as brown oil that solidified upon standing; LC-MS (UV peak area/ESI) 51.2%, 278.0628 [MH⁺] and 48.8%, 228.0425 [MH⁺].

### b) 6-Chloro-5-(cyclopropylmethoxy)-N-[3-(methylcarbamoyl)pentan-3-yl]pyridine-2-carboxamide

A solution of the mixture of 6-(cyclopropylmethoxy)-5-(trifluoromethoxy)pyridine-2-carboxylic acid and 6-chloro-5-(cyclopropylmethoxy)pyridine-2-carboxylic acid (Example 17a, 50 mg), 2-amino-2-ethyl-N-methyl- butanamide (CAN 1415898-90-6, 0.198 mmol), DIEA (154 µL, 0.9 mmol) and TBTU (63.7 mg, 0.198 mmol) in DMF (2 mL) was stirred for 20 h at room temperature. The crude reaction mixture was concentrated in vacuo and purified by flash chromatography (silica gel, 0% to 100% ethyl acetate in heptane) to give the title compound (18 mg, 28%) as off-white solid; LC-MS (UV peak area/ESI) 98%, 354.1581 [MH+].

### Example 18

### 6-Chloro-5-(cyclopropylmethoxy)-N-[(1S)-2-cyclopropyl-1-(5-methyl-1,2,4-oxadiazol-3-yl)ethyl]pyridine-2-carboxamide

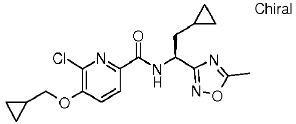

A solution of the mixture of 6-(cyclopropylmethoxy)-5-(trifluoromethoxy)pyridine-2-carboxylic acid and 6-chloro-5-(cyclopropylmethoxy)pyridine-2-carboxylic acid (Example 16a, 50 mg), (α*S*)-α-(cyclopropylmethyl)-5-methyl-1,2,4-oxadiazole-3-methanamine (CAN 1415898-68-8, 0.198 mmol), DIEA (154 µL, 0.9 mmol) and TBTU (63.7 mg, 0.198 mmol) in DMF (2 mL) was stirred for 20 h at room temperature. The crude reaction mixture was concentrated in vacuo and purified by flash chromatography (silica gel, 0% to 100% ethyl acetate in heptane) to give the title compound (20 mg, 29%) as yellow oil; LC-MS (UV peak area/ESI) 89%, 377.1377 [MH+].

### Example 19

### 3-[6-(Cyclopropylmethoxy)-5-(3-methoxyazetidin-1-yl)pyridine-2-carbonyl]-1,1-dioxo-1,3-thiazolidine-4-carboxamide

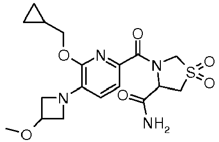

### a) 3-tert-Butoxycarbonyl-1,1-dioxo-1,3-thiazolidine-4-carboxylic acid methyl ester

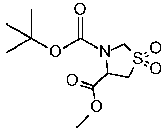

*m*-CPBA (698 mg, 4.04 mmol) was added to an ice cold solution of O3-tert-butyl O4-methyl thiazolidine-3,4-dicarboxylate (CAN 63664-10-8, 0.5 g, 2.02 mmol) in dichloromethane (4 mL). The suspension was stirred for 2 h at ambient temperature. Additional *m*-CPBA (349 mg, 2.02 mmol) was added and stirring was continued over night at ambient temperature. The reaction mixture was poured onto ice water / saturated aqueous NaHCO₃-solution (50 mL). The layers were separated and the aqueous layer was extracted with dichloromethane (2 x 50 mL). The combined organic layers were washed with icewater / brine (30 mL), dried over Na₂SO₄ and concentrated *in vacuo* to give a yellow oil which was purified by column chromatography over silica gel (heptane / AcOEt 0-20 % in 120 min) to obtain the title compound (362 mg, 64%) as colorless oil, MS (ESI) 180.1 [MH-Boc⁺].

### b) 3-tert- Butoxycarbonyl-1,1-dioxo-1,3-thiazolidine-4-carboxylic acid

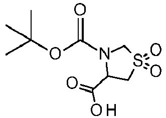

A solution of 3-*tert*-butoxycarbonyl-1,1-dioxo-1,3-thiazolidine-4-carboxylic acid methyl ester (example 19a, 0.35 g, 1.25 mmol) and lithium hydroxide hydrate (63.1 mg, 1.5 mmol) in THF (3.5 mL) and water (1.05 mL) was stirred for 20 h at ambient temperature. The reaction mixture was poured onto ice/0.1N HCl (25 mL) and extracted with EtOAc (2 x 25 mL). The combined extracts were washed with ice / brine (25 mL), dried over Na₂SO₄ and filtered. Removal of the solvent under reduced pressure provided the title compound (306 mg, 92%) as colorless foam, MS (ESI) 264.05 [M-H⁻].

### c) tert-Butyl 4-carbamoyl-1,1-dioxo-1,3-thiazolidine-3-carboxylate

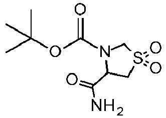

Carbonyldiimidazole (520 mg, 3.21 mmol) was added to an ice cold suspension of 3-*tert-*butoxycarbonyl-1,1-dioxo-1,3-thiazolidine-4-carboxylic acid (example 19b, 304 mg, 1.15 mmol) in DMF (1 mL). The mixture was stirred for 2 h at ambient temperature. Under cooling using a water bath NH₃ gas was bubbled at ambient temperature for 10 min through the solution. Stirring was continued over night at ambient temperature. The mixture was poured into 30 mL ice/water/1N HCl and extracted with EtOAc (2 x 30 mL). The combined extracts were washed with ice / brine (20 mL), dried over Na₂SO₄ and concentrated *in vacuo* to give the title compound (197 mg, 65%) as white solid, MS (ESI) 263.1 [M-H⁻].

### d) 1,1-Dioxo-1,3-thiazolidine-4-carboxamide hydrochloride

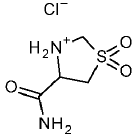

A 4 M solution of hydrogen chloride in dioxane (4.73 mL, 18.9 mmol) was added to an ice cold solution of *tert*-butyl 4-carbamoyl-1,1-dioxo-1,3-thiazolidine-3-carboxylate (example 19c, 500 mg, 1.89 mmol) in dichloromethane (10 mL). The Mixture was stirred for 4 d at ambient temperature. The solvent was removed under reduced pressure to give the title compound (388 mg, quant.) as white solid, MS (ESI): 198.99 [M-H⁻].

### e) 3-[6-(Cyclopropylmethoxy)-5-(3-methoxyazetidin-1-yl)pyridine-2-carbonyl]-1,1-dioxo-1,3-thiazolidine-4-carboxamide

A solution of 6-(cyclopropylmethoxy)-5-(3-methoxyazetidin-1-yl)pyridine-2-carboxylic acid (example 8a, 30 mg, 108 µmol), 1,1-dioxo-1,3-thiazolidine-4-carboxamide hydrochloride (example 19d, 26 mg, 129 µmol), 2-bromo-1-ethylpyridinium tetrafluoroborate (33 mg, 119 µmol) and DIEA (42 mg, 55 µL, 323 µmol) in THF (3 mL) was stirred for 24 h at ambient temperature. Evaporation provided a yellow oil which was dissolved in ice cold saturated aqueous NaHCO₃ solution (75 mL) and ethyl acetate (75 mL). The layers were separated and the aqueous layer was extracted with EtOAc (75 mL). The combined extracts were washed with ice water / 0.1 N HCl (75 mL) and ice water / brine (75 mL), dried over Na₂SO₄ and filtered. After removal of the solvent a yellow solid was obtained which was re-crystallized from EtOAc to obtain the title compound (15 mg, 33%) as off-white solid, MS (ISP): 425.5 [MH⁺].

### Example 20

### Ethyl 2-[[6-(cyclopropylmethoxy)-5-(3-methoxyazetidin-1-yl)pyridine-2-carbonyl]amino]-2-ethylbutanoate

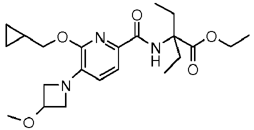

A solution of 6-(cyclopropylmethoxy)-5-(3-methoxyazetidin-1-yl)pyridine-2-carboxylic acid (example 8a, 20.4 mg, 73.3 µmol), DIPEA (23.7 mg, 32.0 µL, 183 µmol) and 4-(4,6-dimethoxy-1,3,5-triazin-2-yl)-4-methylmorpholin-4-ium chloride (22.3 mg, 277 µmol) in dichloromethane (1 mL) was stirred for 30 min at ambient temperature. Ethyl 2-amino-2-ethyl-butanoate hydrochloride (CAN 1135219-29-2, 14.3 mg, 73.3 µmol) was added and the reaction mixture was stirred over night at ambient temperature. Dichloromethane (8 mL) was added and the mixture was washed with 1 M aq. NaHCO₃ solution (3 x 10 mL), water (10 mL) and brine (15 mL). The organic phase was dried over MgSO₄, filtered and the solvent was removed under reduced pressure. Flash chromatography over a 10 g SiO₂ column using heptane:EtOAc (4:1) provided the title compound (25.6 mg, 83.2%) as colorless oil, LC-MS (ESI): 420.7 [MH⁺].

### Example 21

### 6-(Cyclopropylmethoxy)-N-[1-[5-(4-fluorophenyl)-1,3,4-oxadiazol-2-yl]-2-methylpropan-2-yl]-5-(3-methoxyazetidin-1-yl)pyridine-2-carboxamide

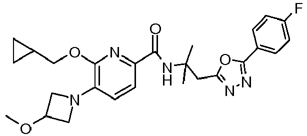

### a) tert-Butyl 4-(2-(4-fluorobenzoyl)hydrazinyl)-2-methyl-4-oxobutan-2-ylcarbamate

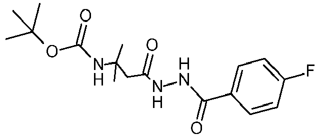

4-Fluorobenzohydrazide (CAN 456-06-4, 2.13 g, 13.8 mmol) was added to a solution of 3-(tert-butoxycarbonylamino)-3-methylbutanoic acid (CAN 129765-95-3, 3 g, 13.8 mmol), DIPEA (5.35 g, 7.23 mL, 41.4 mmol) and HBTU (5.24 g, 13.8 mmol) in DMF (100 mL). The reaction mixture was stirred for 18 h at ambient temperature and subsequently concentrated *in vacuo.* EtOAc (150 mL) was added and the solution was washed with sat. aqueous NaHCO₃ (2 x 50 mL), 1 M HCl (2 x 50 mL) and brine (2 x 50 mL). The aqueous layer was back-extracted with EtOAc (100 mL). The organic layers were combined, dried over MgSO₄ and concentrated *in vacuo.* The crude material was purified by chromatography (silica gel, 300 g, EtOAc:heptane 1:1) to obtain the title compound (1.29 g, 26%) as colorless oil, MS (ISP): 354.3 [MH⁺].

### b) tert-Butyl N-[2-[5-(4-fluorophenyl)-1,3,4-oxadiazol-2-yl]-1,1-dimethyl-ethyl]carbamate

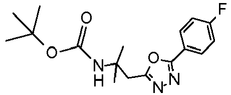

DIPEA (1.42 g, 1.91 mL, 11.0 mmol) and hexachloroethan (1.12 g, 4.75 mmol) were added to a solution of tert-butyl 4-(2-(4-fluorobenzoyl)hydrazinyl)-2-methyl-4-oxobutan-2-ylcarbamate (example 21a, 1.29 g, 3.65 mmol) and triphenylphosphine (1.44 g, 5.48 mmol) in acetonitrile (60 mL). The reaction mixture was stirred for 18 h at ambient temperature and then concentrated *in vacuo.* Dichloromethane (100 mL) was added and the mixture was washed with water (50 mL) and brine (50 mL). The aqueous layer was back-extracted with dichloromethane (100 mL). The combined extracts were dried over MgSO₄ and concentrated *in vacuo.* The crude material was purified by flash chromatography (silica gel, 50 g, 0% to 100% EtOAc in heptane) to obtain the title compound (0.988 g, 81%) as white solid, MS (ISP): 336.3 [MH⁺].

### c) 1-[5-(4-Fluorophenyl)-1,3,4-oxadiazol-2-yl]-2-methyl-propan-2-amine hydrochloride

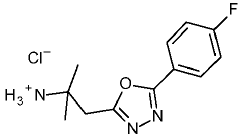

To a solution of tert-butyl *N*-[2-[5-(4-fluorophenyl)-1,3,4-oxadiazol-2-yl]-1,1-dimethylethyl]carbamate (example 21b, 0.98 g, 2.92 mmol) in dioxane (30 mL) was added a 4 M solution of HCl in dioxane (3.65 mL, 14.6 mmol). The reaction mixture was stirred for 5 d at ambient temperature. Additional 4 M HCl in dioxane solution (15 mL) was added and stirring at ambient temperature was continued for 3 d. *t*BuOMe (25 mL) was added, the precipitate was filtered off and dried *in vacuo* to give the title compound (620 mg, 78%), MS (ISP): 236.2 [MH-Cl⁺].

### d) 6-(Cyclopropylmethoxy)-N-[1-[5-(4-fluorophenyl)-1,3,4-oxadiazol-2-yl]-2-methylpropan-2-yl]-5-(3-methoxyazetidin-1-yl)pyridine-2-carboxamide

In analogy to the procedure described in example 20, 6-(cyclopropylmethoxy)-5-(3-methoxyazetidin-1-yl)pyridine-2-carboxylic acid (example 8a, 20.8 mg, 74.7 µmol), was condensed with 1-[5-(4-fluorophenyl)-1,3,4-oxadiazol-2-yl]-2-methyl-propan-2-amine hydrochloride (example 21c, 20.3 mg, 74.7 µmol) to give the title compound (22.8 mg, 62%) as colorless oil, LC-MS (ESI): 496.7 [MH⁺].

### Example 22

### N-[3-(2-Amino-2-oxoethyl)oxetan-3-yl]-6-(cyclopropylmethoxy)-5-(3-methoxyazetidin-1-yl)pyridine-2-carboxamide

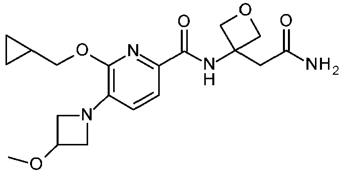

In analogy to the procedure described in example 20, 6-(cyclopropylmethoxy)-5-(3-methoxyazetidin-1-yl)pyridine-2-carboxylic acid (example 8a, 20.2 mg, 72.6 µmol), was condensed with 2-(3-aminooxetan-3-yl)acetamide hydrochloride (CAN of corresponding free base 1417638-25-5, 22.1 mg, 79.8 µmol) to give the title compound (24 mg, 85%) as white powder, LC-MS (ESI): 391.1984 [MH⁺].

### Example 23

### 6-(Cyclopropylmethoxy)-5-(3-methoxyazetidin-1-yl)-N-[3-(methylcarbamoyl)pentan-3-yl]pyridine-2-carboxamide

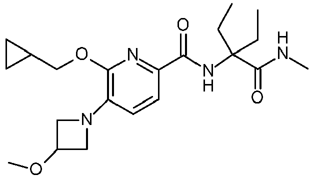

In analogy to the procedure described in example 20, 6-(cyclopropylmethoxy)-5-(3-methoxyazetidin-1-yl)pyridine-2-carboxylic acid (example 8a, 19.7 mg, 70.8 µmol), was condensed with 2-amino-2-ethyl-*N*-methyl-butanamide hydrochloride (CAN 1432507-42-0,21.5 mg, 77.9 µmol) to give the title compound (23.3 mg, 81%) as white powder, LC-MS (ESI): 405.5 [MH⁺].

### Example 24

### 6-(Cyclopropylmethoxy)-N-[(1-hydroxycyclohexyl)methyl]-5-(3-methoxyazetidin-1-yl)pyridine-2-carboxamide

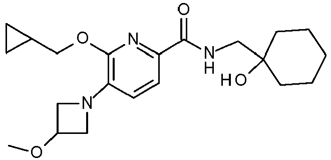

In analogy to the procedure described in example 20, 6-(cyclopropylmethoxy)-5-(3-methoxyazetidin-1-yl)pyridine-2-carboxylic acid (example 8a, 21.7 mg, 78.0 µmol), was condensed with 1-(aminomethyl)cyclohexanol hydrochloride (CAN 19968-85-5, 23.7 mg, 85.8 µmol) to give the title compound (19.8 mg, 65%) as colorless oil, LC-MS (ESI): 390.7 [MH⁺].

### Example 25

### 6-(Cyclopropylmethoxy)-N-[(1-hydroxycyclohexyl)methyl]-5-(3-methoxyazetidin-1-yl)pyridine-2-carboxamide

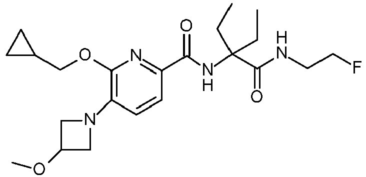

### a) tert-Butyl N-[1-ethyl-1-(2-fluoroethylcarbamoyl)propyl]carbamate

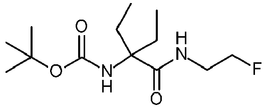

In analogy to the procedure described in example 20, 2-(tert-butoxycarbonylamino)-2-ethyl-butanoic acid (CAN 139937-99-8, 100 mg, 432 µmol) was condensed with 2-fluoroethanamine hydrochloride (CAN 460-08-2, 43.0 mg, 432 µmol) to give the title compound (71 mg, 59%) as white powder, LC-MS (ESI): 277.6 [MH⁺].

### b) N-Ethyl-N-isopropylpropan-2-amine hydrochloride

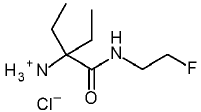

A solution of tert-butyl N-[1-ethyl-1-(2-fluoroethylcarbamoyl)propyl]carbamate (71 mg, 257 µmol) in a 2 M solution of hydrogen chloride (1.03 mL, 2.06 mmol) in diethyl ether was stirred at ambient temperature for 14 h. The solvent was removed under reduced pressure to give crude N-ethyl-N-isopropylpropan-2-amine hydrochloride (63 mg, quant.) which was used in the next reaction step without further purification, LC-MS (ESI): 177.3 [MH⁺] .

### c) 6-(Cyclopropylmethoxy)-N-[(1-hydroxycyclohexyl)methyl]-5-(3-methoxyazetidin-1-yl)pyridine-2-carboxamide

In analogy to the procedure described in example 20, 6-(cyclopropylmethoxy)-5-(3-methoxyazetidin-1-yl)pyridine-2-carboxylic acid (example 8a, 20mg, 71.9 µmol) was condensed with N-ethyl-N-isopropylpropan-2-amine hydrochloride (23.2 mg, 31.4 µL, 180 µmolto give the title compound (21.2 mg, 54%), LC-MS (ESI): 435.3 [M-H⁻].

### Example 26

### 6-(Cyclopropylmethoxy)-N-[3-(3-fluoroazetidine-1-carbonyl)pentan-3-yl]-5-(3-methoxyazetidin-1-yl)pyridine-2-carboxamide

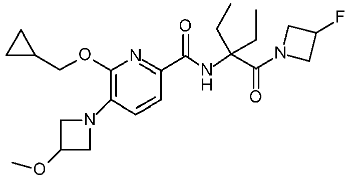

### a) tert-Butyl N-[1-ethyl-1-(3-fluoroazetidine-1-carbonyl)propyl]carbamate

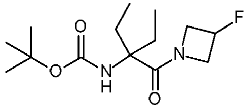

In analogy to the procedure described in example 20, 2-(tert-butoxycarbonylamino)-2-ethyl-butanoic acid (CAN 139937-99-8, 150 mg, 649 µmol) was condensed with 3-fluoroazetidine hydrochloride (CAN 617718-46-4, 72.3 mg, 649 µmol) to give the title compound (72 mg, 39%), LC-MS (ESI): 289.2 [MH⁺].

### b) 2-Amino-2-ethyl-1-(3-fluoroazetidin-1-yl)butan-1-one hydrochloride

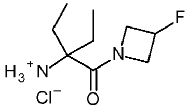

In analogy to the procedure described in example 25 b, tert-butyl N-[1-ethyl-1-(3-fluoroazetidine-1-carbonyl)propyl]carbamate (72mg, 250 µmol) was treated with a 2 M solution of hydrogen chloride in diethyl ether to obtain the title compound (56 mg, quant.) which was used in the next reaction step without further purification, LC-MS (ESI): 189.1 [free amine, M-H⁺].

### c) 6-(Cyclopropylmethoxy)-N-[3-(3-fluoroazetidine-1-carbonyl)pentan-3-yl]-5-(3-methoxyazetidin-1-yl)pyridine-2-carboxamide

In analogy to the procedure described in example 20, 6-(cyclopropylmethoxy)-5-(3-methoxyazetidin-1-yl)pyridine-2-carboxylic acid (example 8a, 20mg, 71.9 µmol) was condensed with 2-amino-2-ethyl-1-(3-fluoroazetidin-1-yl)butan-1-one hydrochloride (16.1 mg, 71.9 µmol) to give the title compound (5.8 mg, 18%), LC-MS (ESI): 449.3 [MH⁺].

### Example 27

### 6-(Cyclopropylmethoxy)-N-[3-(3-fluoropropylcarbamoyl)pentan-3-yl]-5-(3-methoxyazetidin-1-yl)pyridine-2-carboxamide

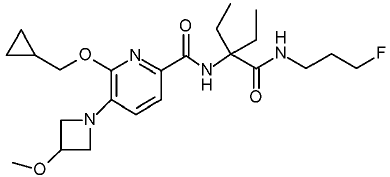

### a) tert-Butyl N-[1-ethyl-1-(3-fluoropropylcarbamoyl)propyl]carbamate

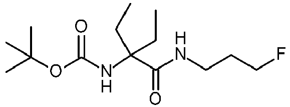

In analogy to the procedure described in example 20, 2-(tert-butoxycarbonylamino)-2-ethyl-butanoic acid (CAN 139937-99-8, 150 mg, 649 µmol) was condensed with 3-fluoropropan-1-amine hydrochloride (CAN 64068-31-1, 73.6 mg, 649 µmol) to give the title compound (128 mg, 68%) as white powder, LC-MS (ESI): 191.2 [M-Boc+2H]⁺.

### b) 2-Amino-2-ethyl-1-(3-fluoroazetidin-1-yl)butan-1-one hydrochloride

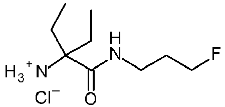

In analogy to the procedure described in example 25 b, tert-butyl N-[1-ethyl-1-(3-fluoropropylcarbamoyl)propyl]carbamate (44mg, 152 µmol) was treated with a 2 M solution of hydrogen chloride in diethyl ether to obtain the title compound (43 mg, quant.) which was used in the next reaction step without further purification, LC-MS (ESI): 191.1 [MH⁺].

### c) 6-(Cyclopropylmethoxy)-N-[3-(3-fluoropropylcarbamoyl)pentan-3-yl]-5-(3-methoxyazetidin-1-yl)pyridine-2-carboxamide

In analogy to the procedure described in example 20, 6-(cyclopropylmethoxy)-5-(3-methoxyazetidin-1-yl)pyridine-2-carboxylic acid (example 8a, 20mg, 71.9 µmol) was condensed with 2-amino-2-ethyl-N-(3-fluoropropyl)butanamide (16.3 mg, 71.9 µmol to give the title compound (17.4 mg, 54%), LC-MS (ESI): 451.5 [MH⁺].

### Example 28

### (2S)-1-[6-(Cyclopropylmethoxy)-5-[3-hydroxy-3-(trifluoromethyl)pyrrolidin-1-yl]pyridine-2-carbonyl]-4,4-difluoropyrrolidine-2-carboxamide

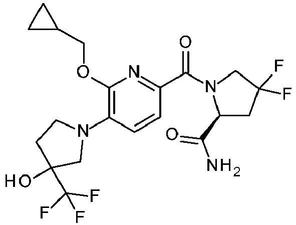

### a) Methyl 6-(cyclopropylmethoxy)-5-[3-hydroxy-3-(trifluoromethyl)pyrrolidin-1-yl]pyridine-2-carboxylate

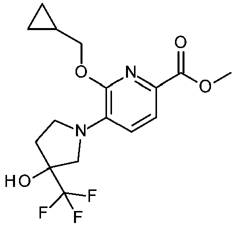

In a 5 mL pear-shaped flask, methyl 5-bromo-6-(cyclopropylmethoxy)pyridine-2-carboxylate (CAN 1415899-20-5, 100 mg, 350 µmol), 3-(trifluoromethyl)pyrrolidin-3-ol hydrochloride (CAN 1334147-81-7, 67 mg, 350 µmol) and caesium carbonate (285 mg, 874 µmol) were combined with toluene (4 mL) to give a white suspension. The mixture was evacuated and purged with argon. Palladium (II) acetate (3.92 mg, 17.5 µmol) and racemic-2,2'-bis(diphenylphosphino)-1,1'-binapthyl (15.2 mg, 24.5 µmol) were added. The reaction mixture was heated to 80 °C and stirred for 2 d. The reaction mixture was diluted with EtOAc and filtered through celite. The filtrate was evaporated to give a yellow oil which was purified by prep. TLC (silica gel, 2.0 mm, heptane/EtOAc 1:1, elution with 100 mL CH₂Cl₂/EtOAc 1:1) to give the title compound (65 mg, 75%) as white solid, MS (ESI): 347.2 [MH⁺].

### b) 6-(Cyclopropylmethoxy)-5-[3-hydroxy-3-(trifluoromethyl)pyrrolidin-1-yl]pyridine-2-carboxylic acid

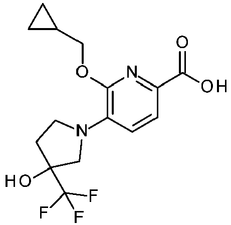

In analogy to the procedure described in example 11 b, methyl 6-(cyclopropylmethoxy)-5-[3-hydroxy-3-(trifluoromethyl)pyrrolidin-1-yl]pyridine-2-carboxylate (95 mg, 264 µmol) was saponified with lithium hydroxide hydrate to give the title compound (84 mg, 92%) as off-white solid, MS (ESI): 347.2 [MH⁺].

### c) (2S)-1-[6-(Cyclopropylmethoxy)-5-[3-hydroxy-3-(trifluoromethyl)pyrrolidin-1-yl]pyridine-2-carbonyl]-4,4-difluoropyrrolidine-2-carboxamide

A mixture of 6-(cyclopropylmethoxy)-5-[3-hydroxy-3-(trifluoromethyl)pyrrolidin-1-yl]pyridine-2-carboxylic acid (15 mg, 43.3 µmol), (S)-4,4-difluoropyrrolidine-2-carboxamide hydrochloride (CAN 426844-51-1, 8.08 mg, 43.3 µmol), 2-bromo-1-ethylpyridinium tetrafluoroborate (13.0 mg, 47.6 µmol) and DIEA (16.8 mg, 22.2 µL, 130 µmol) in THF (1600 µL) was stirred for 1d at ambient temperature, poured onto icewater and acidified to pH 2 with 1N HCl (20 mL). The mixture was extracted with EtOAc (2 x 30 mL) and the organic layers were washed with icewater/brine (20 mL). The organic layers were combined, dried over Na₂SO₄ and concentrated *in vacuo* to give a yellow oil which was purified by prep. HPLC to obtain the title compound (6.2 mg, 30%) as light yellow solid, MS (ESI): 479.3 [MH⁺].

### Example 29

### N-[(2S)-1-Amino-4-methyl-1-oxopentan-2-yl]-6-(cyclopropylmethoxy)-5-[3-hydroxy-3-(trifluoromethyl)pyrrolidin-1-yl]pyridine-2-carboxamide

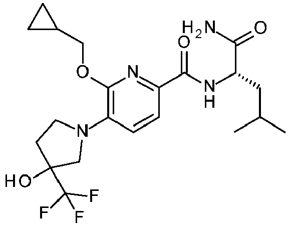

In analogy to the procedure described in example 28 c, 6-(cyclopropylmethoxy)-5-[3-hydroxy-3-(trifluoromethyl)pyrrolidin-1-yl]pyridine-2-carboxylic acid (example 28 b, 15 mg, 43.3 µmol) was condensed with (S)-2-amino-4-methylpentanamide hydrochloride (CAN 687-51-4, 7.22 mg, 43.3 µmol) in the presence of 2-bromo-1-ethylpyridinium tetrafluoroborate and DIEA to give the title compound (15 mg, 76%) as yellow oil, MS (ESI): 459.4 [MH⁺].

### Example 30

### N-[(2S)-1-Amino-4-methyl-1-oxopentan-2-yl]-6-(cyclopropylmethoxy)-5-(2,2-difluoro-5-azaspiro[2.4]heptan-5-yl)pyridine-2-carboxamide

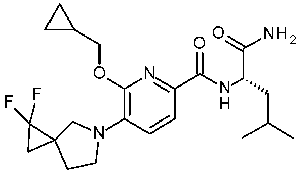

### a) Methyl 6-(cyclopropylmethoxy)-5-(2,2-difluoro-5-azaspiro[2.4]heptan-5-yl)pyridine-2-carboxylate

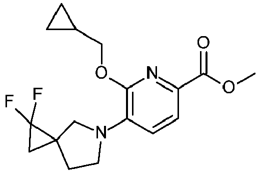

In analogy to the procedure described in example 28 a, methyl 5-bromo-6-(cyclopropylmethoxy)pyridine-2-carboxylate (CAN 1415899-20-5, 70 mg, 246 µmol) was reacted with 1,1-difluoro-5-azaspiro[2.4]heptane hydrochloride (CAN 1215071-12-7, 42 mg, 246 µmol) to give the title compound (65 mg, 78%) as yellow oil, MS (ESI): 339.3 [MH⁺] .

### b) 6-(Cyclopropylmethoxy)-5-(2,2-difluoro-5-azaspiro[2.4]heptan-5-yl)pyridine-2-carboxylic acid

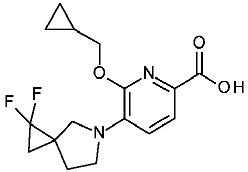

In analogy to the procedure described in example 11 b, methyl 6-(cyclopropylmethoxy)-5-(2,2-difluoro-5-azaspiro[2.4]heptan-5-yl)pyridine-2-carboxylate (69 mg, 204 µmol) was saponified with lithium hydroxide hydrate to give the title compound (67 mg, quant.) as off-white solid, MS (ESI): 325.2 [MH⁺].

### c) N-[(2S)-1-Amino-4-methyl-1-oxopentan-2-yl]-6-(cyclopropylmethoxy)-5-(2,2-difluoro-5-azaspiro[2.4]heptan-5-yl)pyridine-2-carboxamide

In analogy to the procedure described in example 28 c, 6-(cyclopropylmethoxy)-5-(2,2-difluoro-5-azaspiro[2.4]heptan-5-yl)pyridine-2-carboxylic acid (15.3 mg, 47.2 µmol) was condensed with (S)-2-amino-4-methylpentanamide hydrochloride (CAN 687-51-4, 7.86 mg, 47.2 µmol) in the presence of 2-bromo-1-ethylpyridinium tetrafluoroborate and DIEA to give the title compound (11 mg, 53%) as colorless oil, MS (ESI): 437.3 [MH⁺].

### Example 31

### (2S)-1-[6-(Cyclopropylmethoxy)-5-(2,2-difluoro-5-azaspiro[2.4]heptan-5-yl)pyridine-2-carbonyl]-4,4-difluoropyrrolidine-2-carboxamide

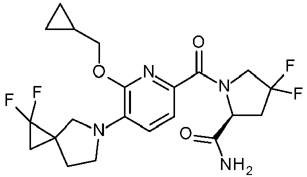

In analogy to the procedure described in example 28 c, 6-(cyclopropylmethoxy)-5-(2,2-difluoro-5-azaspiro[2.4]heptan-5-yl)pyridine-2-carboxylic acid (example 30 b, 15.3 mg, 47.2 µmol) was condensed with (S)-4,4-difluoropyrrolidine-2-carboxamide hydrochloride (CAN 426844-51-1, 8.8 mg, 47.2 µmol) in the presence of 2-bromo-1-ethylpyridinium tetrafluoroborate and DIEA to give the title compound (13 mg, 59%) as white solid, MS (ESI): 457.3 [MH⁺].

### Example 32

### N-[3-(2-Amino-2-oxoethyl)oxetan-3-yl]-6-(cyclopropylmethoxy)-5-(2,2-difluoro-5-azaspiro[2.4]heptan-5-yl)pyridine-2-carboxamide

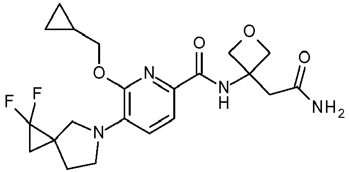

In analogy to the procedure described in example 28 c, 6-(cyclopropylmethoxy)-5-(2,2-difluoro-5-azaspiro[2.4]heptan-5-yl)pyridine-2-carboxylic acid (example 30 b, 15.3 mg, 47.2 µmol) was condensed with 2-(3-aminooxetan-3-yl)acetamide (CAN 1417638-25-5, 6.14 mg, 47.2 µmol) in the presence of 2-bromo-1-ethylpyridinium tetrafluoroborate and DIEA to give the title compound (9 mg, 43%) as white solid, MS (ESI): 437.3 [MH⁺].

### Example 33

### N-[(2S)-1-Amino-4-methyl-1-oxopentan-2-yl]-6-(cyclopropylmethoxy)-5-(3-fluoro-3-methylazetidin-1-yl)pyridine-2-carboxamide

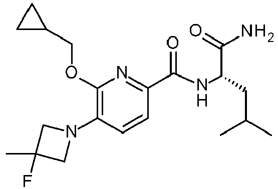

### a) Methyl 6-(cyclopropylmethoxy)-5-(3-fluoro-3-methyl-azetidin-1-yl)pyridine-2-carboxylate

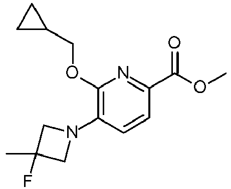

In analogy to the procedure described in example 28 a, methyl 5-bromo-6-(cyclopropylmethoxy)pyridine-2-carboxylate (CAN 1415899-20-5, 59 mg, 207 µmol) was reacted with 3-fluoro-3-methylazetidine hydrochloride (CAN 1427379-42-7, 26 mg, 207 µmol) to give the title compound (53 mg, 87%) as yellow oil, MS (ESI): 295.3 [MH⁺].

### b) 6-(Cyclopropylmethoxy)-5-(3-fluoro-3-methyl-azetidin-1-yl)pyridine-2-carboxylic acid

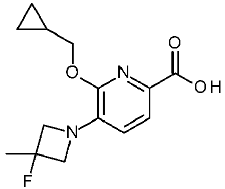

In analogy to the procedure described in example 11 b, methyl 6-(cyclopropylmethoxy)-5-(3-fluoro-3-methyl-azetidin-1-yl)pyridine-2-carboxylate (53.8 mg, 183 µmol) was saponified with lithium hydroxide hydrate to give the title compound (55 mg, quant.) as off-white solid, MS (ESI): 281.2 [MH⁺].

### c) N-[(2S)-1-Amino-4-methyl-1-oxopentan-2-yl]-6-(cyclopropylmethoxy)-5-(3-fluoro-3-methylazetidin-1-yl)pyridine-2-carboxamide

In analogy to the procedure described in example 28 c, 6-(cyclopropylmethoxy)-5-(3-fluoro-3-methyl-azetidin-1-yl)pyridine-2-carboxylic acid (15.3 mg, 54.6 µmol) was condensed with (S)-2-amino-4-methylpentanamide hydrochloride (CAN 687-51-4, 9.1 mg, 54.6 µmol) in the presence of 2-bromo-1-ethylpyridinium tetrafluoroborate and DIEA to give the title compound (19 mg, 87%) as light yellow oil, MS (ESI): 393.3 [MH⁺].

### Example 34

### (2S)-1-[6-(Cyclopropylmethoxy)-5-(3-fluoro-3-methylazetidin-1-yl)pyridine-2-carbonyl]-4,4-difluoropyrrolidine-2-carboxamide

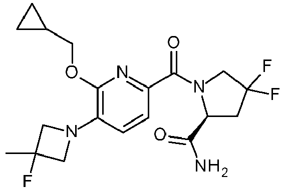

In analogy to the procedure described in example 28 c, 6-(cyclopropylmethoxy)-5-(3-fluoro-3-methyl-azetidin-1-yl)pyridine-2-carboxylic acid (example 33 b, 15.3 mg, 54.6 µmol) was condensed with (S)-4,4-difluoropyrrolidine-2-carboxamide hydrochloride (CAN 426844-51-1, 10.2 mg, 54.6 µmol) in the presence of 2-bromo-1-ethylpyridinium tetrafluoroborate and DIEA to give the title compound (20 mg, 88%) as white solid, MS (ESI): 413.3 [MH⁺].

### Example 35

### N-[3-(2-Amino-2-oxoethyl)oxetan-3-yl]-6-(cyclopropylmethoxy)-5-(3-fluoro-3-methylazetidin-1-yl)pyridine-2-carboxamide

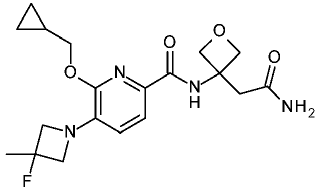

In analogy to the procedure described in example 28 c, 6-(cyclopropylmethoxy)-5-(3-fluoro-3-methyl-azetidin-1-yl)pyridine-2-carboxylic acid (example 33 b, 15.3 mg, 54.6 µmol) was condensed with 2-(3-aminooxetan-3-yl)acetamide (CAN 1417638-25-5, 7.1 mg, 54.6 µmol) in the presence of 2-bromo-1-ethylpyridinium tetrafluoroborate and DIEA to give the title compound (10 mg, 48%) as off white solid, MS (ESI): 393.3 [MH⁺].

### Example 36

### N-[(2S)-1-Amino-4-methyl-1-oxopentan-2-yl]-5-(3-cyclopropyl-3-fluoroazetidin-1-yl)-6-(cyclopropylmethoxy)pyridine-2-carboxamide

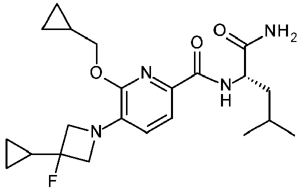

### a) Methyl 5-(3-cyclopropyl-3-fluoro-azetidin-1-yl)-6-(cyclopropylmethoxy)pyridine-2-carboxylate

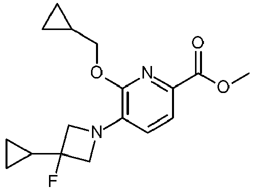

In analogy to the procedure described in example 28 a, methyl 5-bromo-6-(cyclopropylmethoxy)pyridine-2-carboxylate (CAN 1415899-20-5, 60 mg, 210 µmol) was reacted with 3-cyclopropyl-3-fluoroazetidine hydrochloride (CAN 936548-77-5, 31.8 mg, 210 µmol) to give the title compound (61 mg, 91%) as yellow oil, MS (ESI): 321.3 [MH⁺].

### b) 5-(3-Cyclopropyl-3-fluoro-azetidin-1-yl)-6-(cyclopropylmethoxy)pyridine-2-carboxylic acid

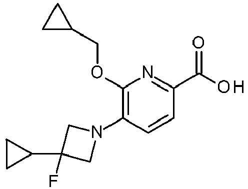

In analogy to the procedure described in example 11 b, methyl 5-(3-cyclopropyl-3-fluoro-azetidin-1-yl)-6-(cyclopropylmethoxy)pyridine-2-carboxylate (56.7 mg, 177 µmol) was saponified with lithium hydroxide hydrate to give the title compound (60 mg, quant.) as off-white solid, MS (ESI): 307.2 [MH⁺].

### c) N-[(2S)-1-Amino-4-methyl-1-oxopentan-2-yl]-5-(3-cyclopropyl-3-fluoroazetidin-1-yl)-6-(cyclopropylmethoxy)pyridine-2-carboxamide

In analogy to the procedure described in example 28 c, 5-(3-cyclopropyl-3-fluoro-azetidin-1-yl)-6-(cyclopropylmethoxy)pyridine-2-carboxylic acid (15.1 mg, 49.3 µmol) was condensed with (S)-2-amino-4-methylpentanamide hydrochloride (CAN 687-51-4, 8.21 mg, 49.3 µmol) in the presence of 2-bromo-1-ethylpyridinium tetrafluoroborate and DIEA to give the title compound (15 mg, 72%) as colorless oil, MS (ESI): 419.3 [MH⁺].

### Example 37

### (2S)-1-[5-(3-Cyclopropyl-3-fluoroazetidin-1-yl)-6-(cyclopropylmethoxy)pyridine-2-carbonyl]-4,4-difluoropyrrolidine-2-carboxamide

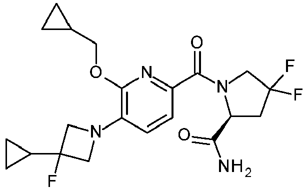

In analogy to the procedure described in example 28 c, 5-(3-cyclopropyl-3-fluoro-azetidin-1-yl)-6-(cyclopropylmethoxy)pyridine-2-carboxylic acid (example 36 b, 15.1 mg, 49.3 µmol) was condensed with (S)-4,4-difluoropyrrolidine-2-carboxamide hydrochloride (CAN 426844-51-1, 9.2 mg, 49.3 µmol) in the presence of 2-bromo-1-ethylpyridinium tetrafluoroborate and DIEA to give the title compound (21 mg, 95%) as white solid, MS (ESI): 439.3 [MH⁺].

### Example 38

### N-[3-(2-Amino-2-oxoethyl)oxetan-3-yl]-5-(3-cyclopropyl-3-fluoroazetidin-1-yl)-6-(cyclopropylmethoxy)pyridine-2-carboxamide

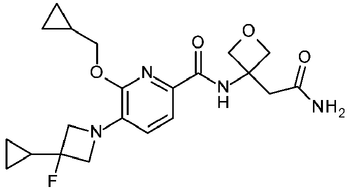

In analogy to the procedure described in example 28 c, 5-(3-cyclopropyl-3-fluoro-azetidin-1-yl)-6-(cyclopropylmethoxy)pyridine-2-carboxylic acid (example 36 b, 15.1 mg, 49.3 µmol) was condensed with 2-(3-aminooxetan-3-yl)acetamide (CAN 1417638-25-5, 6.42 mg, 49.3 µmol) in the presence of 2-bromo-1-ethylpyridinium tetrafluoroborate and DIEA to give the title compound (12 mg, 56%) as colorless oil, MS (ESI): 419.3 [MH⁺].

### Example 39

### (2S)-1-[6-(4-Chlorophenyl)-5-(cyclopropylmethoxy)pyridine-2-carbonyl]-4,4-difluoropyrrolidine-2-carboxamide

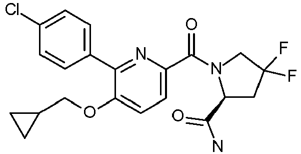

In analogy to the procedure described in example 28 c, 6-(4-chlorophenyl)-5-(cyclopropylmethoxy)pyridine-2-carboxylic acid (CAN 1364677-94-0, 26 mg, 86 µmol) was condensed with (S)-4,4-difluoropyrrolidine-2-carboxamide hydrochloride (CAN 426844-51-1, 16 mg, 86 µmol) in the presence of 2-bromo-1-ethylpyridinium tetrafluoroborate and DIEA to give the title compound (15 mg, 39%) as white solid, LC-MS (ESI): 436.1249.

### Example 40

### 6-(4-Chlorophenyl)-5-(cyclopropylmethoxy)-N-[2-(5-methyl-1,2,4-oxadiazol-3-yl)propan-2-yl]pyridine-2-carboxamide

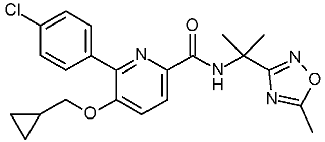

In analogy to the procedure described in example 28 c, 6-(4-chlorophenyl)-5-(cyclopropylmethoxy)pyridine-2-carboxylic acid (CAN 1364677-94-0, 34 mg, 112 µmol) was condensed with 2-(5-methyl-1,2,4-oxadiazol-3-yl)propan-2-amine hydrochloride (CAN 1240526-27-5, 19.9 mg, 112 µmol) in the presence of 2-bromo-1-ethylpyridinium tetrafluoroborate and DIEA to give the title compound (46 mg, 96%) as light orange oil, MS (ESI): 427.2 [MH⁺].

### Example 41

### 5-(3-Cyclopropyl-3-fluoroazetidin-1-yl)-6-(cyclopropylmethoxy)-N-[3-(3-fluoropropylcarbamoyl)pentan-3-yl]pyridine-2-carboxamide

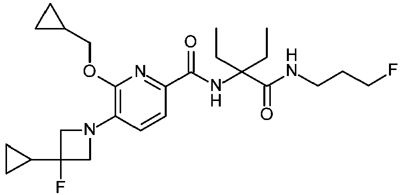

In analogy to the procedure described in example 28 c, 5-(3-cyclopropyl-3-fluoro-azetidin-1-yl)-6-(cyclopropylmethoxy)pyridine-2-carboxylic acid (example 36 b, 10 mg, 33 µmol) was condensed with 2-amino-2-ethyl-N-(3-fluoropropyl)butanamide hydrochloride (CAN 1613239-88-5, 8 mg, 36 µmol) in the presence of 2-bromo-1-ethylpyridinium tetrafluoroborate and DIEA to give the title compound (5 mg, 32%) as white solid, MS (ESI): m/e = 479.3 [MH⁺].

### Example 42

### N-[(2S)-1-Amino-4-methyl-1-oxopentan-2-yl]-6-(4-chlorophenyl)-5-(cyclopropylmethoxy)pyridine-2-carboxamide

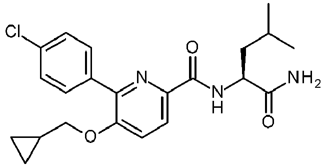

In analogy to the procedure described in example 28 c, 6-(4-chlorophenyl)-5-(cyclopropylmethoxy)pyridine-2-carboxylic acid (CAN 1364677-94-0, 35 mg, 115 µmol) was condensed with (S)-2-amino-4-methylpentanamide hydrochloride (CAN 687-51-4, 19 mg, 115 µmol) in the presence of 2-bromo-1-ethylpyridinium tetrafluoroborate and DIEA to give the title compound (30 mg, 63%) as white solid, MS (ESI): 416.2 [MH⁺].

### Example 43

### Pharmacological tests

The following tests were carried out in order to determine the activity of the compounds of formula I:

### Radioligand binding assay

The affinity of the compounds of the invention for cannabinoid CB1 receptors was determined using recommended amounts of membrane preparations (PerkinElmer) of human embryonic kidney (HEK) cells expressing the human CNR1 or CNR2 receptors in conjunction with 1.5 or 2.6 nM [3H]-CP-55,940 (Perkin Elmer) as radioligand, respectively. Binding was performed in binding buffer (50 mM Tris, 5 mM MgCl2, 2.5 mM EDTA, and 0.5% (wt/vol) fatty acid free BSA, pH 7.4 for CB1 receptor and 50 mM Tris, 5 mM MgCl2, 2.5 mM EGTA, and 0.1% (wt/vol) fatty acid free BSA, pH 7.4 for CB2 receptor) in a total volume of 0.2 ml for 1h at 30°C shaking. The reaction was terminated by rapid filtration through microfiltration plates coated with 0.5% polyethylenimine (UniFilter GF/B filter plate; Packard). Bound radioactivity was analyzed for Ki using nonlinear regression analysis (Activity Base, ID Business Solution, Limited), with the Kd values for [3H]CP55,940 determined from saturation experiments. The compounds of formula (I) show an excellent affinity for the CB2 receptor.

The compounds according to formula (I) have an activity in the above assay (Ki) between 0.5 nM and 10 µM. Particular compounds of formula (I) have an activity in the above assay (Ki) between 0.5 nM and 3 µM. Other particular compounds of formula (I) have an activity in the above assay (Ki) between 0.5 nM and 100 nM.

### cAMP Assay

CHO cells expressing human CB1 or CB2 receptors are seeded 17-24 hours prior to the experiment 50.000 cells per well in a black 96 well plate with flat clear bottom (Corning Costar #3904) in DMEM (Invitrogen No. 31331), 1x HT supplement, with 10 % fetal calf serum and incubated at 5% CO₂ and 37°C in a humidified incubator. The growth medium was exchanged with Krebs Ringer Bicarbonate buffer with 1 mM IBMX and incubated at 30°C for 30 min. Compounds were added to a final assay volume of 100 µl and incubated for 30 min at 30°C. Using the cAMP-Nano-TRF detection kit the assay (Roche Diagnostics) was stopped by the addition of 50 µl lysis reagent (Tris, NaCl, 1.5% Triton X100, 2.5% NP40, 10% NaN₃) and 50 µl detection solutions (20 µM mAb Alexa700-cAMP 1:1, and 48 µM Ruthenium-2-AHA-cAMP) and shaken for 2h at room temperature. The time-resolved energy transfer is measured by a TRF reader (Evotec Technologies GmbH), equipped with a ND:YAG laser as excitation source. The plate is measured twice with the excitation at 355 nm and at the emission with a delay of 100 ns and a gate of 100 ns, total exposure time 10s at 730 (bandwidth 30 nm) or 645 nm (bandwidth 75 nm), respectively. The FRET signal is calculated as follows: FRET = T730-Alexa730-P(T645-B645) with P = Ru730-B730/Ru645-B645, where T730 is the test well measured at 730 nM, T645 is the test well measured at 645 nm, B730 and B645 are the buffer controls at 730 nm and 645 nm, respectively. cAMP content is determined from the function of a standard curve spanning from 10 µM to 0.13 nM cAMP.

EC₅₀ values were determined using Activity Base analysis (ID Business Solution, Limited). The EC₅₀ values for a wide range of cannabinoid agonists generated from this assay for reference compounds were in agreement with the values published in the scientific literature.

In the foregoing assay, the compounds according to the invention have a human CB2 EC₅₀ which is between 0.5 nM and 10 µM. Particular compounds according to the invention have a human CB2 EC₅₀ between 0.5 nM and 1 µM. Further particular compounds according to the invention have a human CB2 EC₅₀ between 0.5 nM and 100 nM. They exhibit at least 10 fold selectivity against the human CB1 receptor in, either both of the radioligand and cAMP assay, or in one of these two assays.

Results obtained for representative compounds of the invention are given in the following table.

| **Example** | cAMP assay human CB2 EC₅₀ [µM] |
|---|---|
| **1** | 0.3436 |
| **2** | 0.0482 |
| **3** | 0.1439 |
| **4** | 0.2316 |
| **5** | 0.0342 |
| **6** | 0.8309 |
| **7** | 0.0513 |
| **8** | 0.0866 |
| **9** | 0.0424 |
| **10** | 0.0634 |
| **11** | 0.1352 |
| **12** | 0.1765 |
| **13** | 0.0231 |
| **14** | 0.095 |
| **15** | 0.0219 |
| **16** | 0.9731 |
| **17** | 0.7996 |
| **18** | 0.6456 |
| **19** | 0.0302 |
| **20** | 0.0065 |
| **21** | 0.853 |
| **22** | 0.067 |
| **23** | 0.047 |
| **24** | 1.291 |
| **25** | 0.33427 |
| **26** | 0.00458 |
| **27** | 0.44432 |
| **28** | 1.68031 |
| **29** | 1.88566 |
| **30** | 0.03813 |
| **31** | 0.01904 |
| **32** | 0.06025 |
| **33** | 0.01955 |
| **34** | 0.01138 |
| **35** | 0.21321 |
| **36** | 0.01998 |
| **37** | 0.01935 |
| **38** | 0.04833 |
| **39** | 0.01805 |
| **40** | 0.01689 |
| **41** | 0.669 |
| **42** | 0.00444 |

### Example A

Film coated tablets containing the following ingredients can be manufactured in a conventional manner:

| Ingredients | Per tablet | |
|---|---|---|
| **Kernel:** | | |
| Compound of formula (I) | 10.0 mg | 200.0 mg |
| Microcrystalline cellulose | 23.5 mg | 43.5 mg |
| Lactose hydrous | 60.0 mg | 70.0 mg |
| Povidone K30 | 12.5 mg | 15.0 mg |
| Sodium starch glycolate | 12.5 mg | 17.0 mg |
| Magnesium stearate | 1.5 mg | 4.5 mg |
| (Kernel Weight) | 120.0 mg | 350.0 mg |

| **Film Coat:** | | |
|---|---|---|
| Hydroxypropyl methyl cellulose | 3.5 mg | 7.0 mg |
| Polyethylene glycol 6000 | 0.8 mg | 1.6 mg |
| Talc | 1.3 mg | 2.6 mg |
| Iron oxide (yellow) | 0.8 mg | 1.6 mg |
| Titan dioxide | 0.8 mg | 1.6 mg |

The active ingredient is sieved and mixed with microcrystalline cellulose and the mixture is granulated with a solution of polyvinylpyrrolidone in water. The granulate is then mixed with sodium starch glycolate and magnesium stearate and compressed to yield kernels of 120 or 350 mg respectively. The kernels are lacquered with an aq. solution / suspension of the above mentioned film coat.

### Example B

Capsules containing the following ingredients can be manufactured in a conventional manner:

| Ingredients | Per capsule |
|---|---|
| Compound of formula (I) | 25.0 mg |
| Lactose | 150.0 mg |
| Maize starch | 20.0 mg |
| Talc | 5.0 mg |

The components are sieved and mixed and filled into capsules of size 2.

### Example C

Injection solutions can have the following composition:

| | |
|---|---|
| Compound of formula (I) | 3.0 mg |
| Polyethylene glycol 400 | 150.0 mg |
| Acetic acid | q.s. ad pH 5.0 |
| Water for injection solutions | ad 1.0 ml |

The active ingredient is dissolved in a mixture of Polyethylene glycol 400 and water for injection (part). The pH is adjusted to 5.0 by addition of acetic acid. The volume is adjusted to 1.0 ml by addition of the residual amount of water. The solution is filtered, filled into vials using an appropriate overage and sterilized.

## Claims

1. A compound of formula (I) 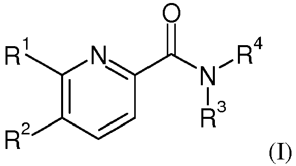 wherein
R¹ is alkylsulfanyl, cycloalkylalkoxy, halophenyl or halogen;
R² is phenyl, halophenyl, cycloalkylalkoxy, alkoxyazetidinyl, 2-oxa-6-azaspiro[3.3]heptyl, (haloalkyl)(hydroxy)pyrrolidinyl, halo-5-azaspiro[2.4]heptyl, (alkyl)(halo)azetidinyl or (cycloalkyl)(halo)azetidinyl;
one of R³ and R⁴ is hydrogen and the other one is -(CR⁵R⁶)ₘ-(CH₂)ₙ-R⁷;
or R³ and R⁴ together with the nitrogen atom to which they are attached form aminocarbonyl-dioxo-thiazolidinyl or (aminocarbonyl)(halo)pyrrolidinyl;
R⁵ and R⁶ are independently selected from hydrogen, alkyl, cycloalkylalkyl and alkylsulfanylalkyl;
or R⁵and R⁶ together with the carbon atom to which they are attached form oxetanyl;
R⁷ is alkoxycarbonyl, oxazol-2-yl, 5-alkyl-1,2,4-oxadiazol-3-yl, aminocarbonyl, alkylaminocarbonyl, thiazol-2-yl, 5-halophenyl-1,3,4-oxadiazol-2-yl or hydroxycycloalkyl, haloalkylaminocarbonyl or haloazetidinylcarbonyl;
m is 0 or 1;
n is 0 or 1;
or a pharmaceutically acceptable salt or ester thereof.

2. A compound according to claim 1, wherein R¹ is cycloalkylalkoxy or halophenyl.

3. A compound according to claim 1 or 2, wherein R¹ is cyclopropylmethoxy or chlorophenyl.

4. A compound according to any one of claims 1 to 3, wherein R² is phenyl, halophenyl, cycloalkylalkoxy or alkoxyazetidinyl.

5. A compound according to any one of claims 1 to 4, wherein R² is phenyl, chlorophenyl, cyclopropylmethoxy or methoxyazetidinyl.

6. A compound according to any one of claims 1 to 5, wherein R⁵ and R⁶ are independently selected from hydrogen and alkyl.

7. A compound according to any one of claims 1 to 6, wherein R⁵ and R⁶ are independently selected from hydrogen, methyl, ethyl and isobutyl.

8. A compound according to any one of claims 1 to 7, wherein R⁷ is alkoxycarbonyl, oxazol-2-yl, thiazol-2-yl or aminocarbonyl.

9. A compound according to any one of claims 1 to 8, wherein R⁷ is methoxycarbonyl, oxazol-2-yl, thiazol-2-yl or aminocarbonyl.

10. A compound according to any one of claims 1 to 9 selected from
Methyl 2-[[5-(4-chlorophenyl)-6-methylsulfanylpyridine-2-carbonyl] amino]-2-methylpropanoate;
Methyl 2-[[5-(4-chlorophenyl)-6-(cyclopropylmethoxy)pyridine-2-carbonyl] amino]-2-methylpropanoate;
5-(4-Chlorophenyl)-6-(cyclopropylmethoxy)-*N*-[2-(1,3-oxazol-2-yl)propan-2-yl]pyridine-2-carboxamide;
5-(4-Chlorophenyl)-6-(cyclopropylmethoxy)-*N*-[2-(5-methyl-1,2,4-oxadiazol-3-yl)propan-2-yl]pyridine-2-carboxamide;
6-(Cyclopropylmethoxy)-*N*-[2-(1,3-oxazol-2-yl)propan-2-yl]-5-phenylpyridine-2-carboxamide;
6-(Cyclopropylmethoxy)-*N*-[3-(methylcarbamoyl)pentan-3-yl]-5-phenylpyridine-2-carboxamide;
6-(3-Chlorophenyl)-5-(cyclopropylmethoxy)-*N*-[3-(methylcarbamoyl)pentan-3-yl]pyridine-2-carboxamide;
6-(Cyclopropylmethoxy)-5-(3-methoxyazetidin-1-yl)-*N*-[2-(5-methyl-1,2,4-oxadiazol-3-yl)propan-2-yl]pyridine-2-carboxamide;
6-(3-Chlorophenyl)-5-(cyclopropylmethoxy)-*N*-[2-(5-methyl-1,2,4-oxadiazol-3-yl)propan-2-yl]pyridine-2-carboxamide;
*N*-[(2*S*)-1-amino-3-cyclopropyl-1-oxopropan-2-yl]-6-(cyclopropylmethoxy)-5-(3-methoxyazetidin-1-yl)pyridine-2-carboxamide;
6-(Cyclopropylmethoxy)-5-(2-oxa-6-azaspiro[3.3]heptan-6-yl)-*N*-[2-(1,3-thiazol-2-yl)propan-2-yl]pyridine-2-carboxamide;
*N*-[(2*S*)-1-amino-4-methyl-1-oxopentan-2-yl]-6-(cyclopropylmethoxy)-5-(2-oxa-6-azaspiro[3.3]heptan-6-yl)pyridine-2-carboxamide;
6-(3-Chlorophenyl)-5-(cyclopropylmethoxy)-*N*-[2-(1,3-thiazol-2-yl)propan-2-yl]pyridine-2-carboxamide;
*N*-[(2*S*)-1-amino-3-cyclopropyl-1-oxopropan-2-yl]-5,6-bis(cyclopropylmethoxy)pyridine-2-carboxamide; *N*-[(2*S*)-1-amino-4-methyl-1-oxopentan-2-yl]-6-(cyclopropylmethoxy)-5-(3-methoxyazetidin-1-yl)pyridine-2-carboxamide;
*N*-[(2*S*)-1-amino-4-methylsulfanyl-1-oxobutan-2-yl]-6-chloro-5-(cyclopropylmethoxy)pyridine-2-carboxamide;
6-Chloro-5-(cyclopropylmethoxy)-*N*-[3-(methylcarbamoyl)pentan-3-yl]pyridine-2-carboxamide;
6-Chloro-5-(cyclopropylmethoxy)-*N*-[(1*S*)-2-cyclopropyl-1-(5-methyl-1,2,4-oxadiazol-3-yl)ethyl]pyridine-2-carboxamide;
3-[6-(Cyclopropylmethoxy)-5-(3-methoxyazetidin-1-yl)pyridine-2-carbonyl]-1,1-dioxo-1,3-thiazolidine-4-carboxamide;
Ethyl 2-[[6-(cyclopropylmethoxy)-5-(3-methoxyazetidin-1-yl)pyridine-2-carbonyl] amino] -2-ethylbutanoate;
6-(Cyclopropylmethoxy)-*N*-[1-[5-(4-fluorophenyl)-1,3,4-oxadiazol-2-yl]-2-methylpropan-2-yl]-5-(3-methoxyazetidin-1-yl)pyridine-2-carboxamide;
*N*-[3-(2-Amino-2-oxoethyl)oxetan-3-yl]-6-(cyclopropylmethoxy)-5-(3-methoxyazetidin-1-yl)pyridine-2-carboxamide;
6-(Cyclopropylmethoxy)-5-(3-methoxyazetidin-1-yl)-N-[3-(methylcarbamoyl)pentan-3-yl]pyridine-2-carboxamide;
6-(Cyclopropylmethoxy)-N-[(1-hydroxycyclohexyl)methyl]-5-(3-methoxyazetidin-1-yl)pyridine-2-carboxamide;
6-(Cyclopropylmethoxy)-N-[3-(2-fluoroethylcarbamoyl)pentan-3-yl]-5-(3-methoxyazetidin-1-yl)pyridine-2-carboxamide;
6-(Cyclopropylmethoxy)-N-[3-(3-fluoroazetidine-1-carbonyl)pentan-3-yl]-5-(3-methoxyazetidin-1-yl)pyridine-2-carboxamide;
6-(Cyclopropylmethoxy)-N-[3-(3-fluoropropylcarbamoyl)pentan-3-yl]-5-(3-methoxyazetidin-1-yl)pyridine-2-carboxamide;
(2S)-1-[6-(Cyclopropylmethoxy)-5-[3-hydroxy-3-(trifluoromethyl)pyrrolidin-1-yl]pyridine-2-carbonyl]-4,4-difluoropyrrolidine-2-carboxamide;
N-[(2S)-1-Amino-4-methyl-1-oxopentan-2-yl]-6-(cyclopropylmethoxy)-5-[3-hydroxy-3-(trifluoromethyl)pyrrolidin-1-yl]pyridine-2-carboxamide;
N-[(2S)-1-Amino-4-methyl-1-oxopentan-2-yl]-6-(cyclopropylmethoxy)-5-(2,2-difluoro-5-azaspiro[2.4]heptan-5-yl)pyridine-2-carboxamide;
(2S)-1-[6-(Cyclopropylmethoxy)-5-(2,2-difluoro-5-azaspiro[2.4]heptan-5-yl)pyridine-2-carbonyl]-4,4-difluoropyrrolidine-2-carboxamide;
N-[3-(2-Amino-2-oxoethyl)oxetan-3-yl]-6-(cyclopropylmethoxy)-5-(2,2-difluoro-5-azaspiro[2.4]heptan-5-yl)pyridine-2-carboxamide;
N-[(2S)-1-Amino-4-methyl-1-oxopentan-2-yl]-6-(cyclopropylmethoxy)-5-(3-fluoro-3-methylazetidin-1-yl)pyridine-2-carboxamide;
(2S)-1-[6-(Cyclopropylmethoxy)-5-(3-fluoro-3-methylazetidin-1-yl)pyridine-2-carbonyl]-4,4-difluoropyrrolidine-2-carboxamide;
N-[3-(2-Amino-2-oxoethyl)oxetan-3-yl]-6-(cyclopropylmethoxy)-5-(3-fluoro-3-methylazetidin-1-yl)pyridine-2-carboxamide;
N-[(2S)-1-Amino-4-methyl-1-oxopentan-2-yl]-5-(3-cyclopropyl-3-fluoroazetidin-1-yl)-6-(cyclopropylmethoxy)pyridine-2-carboxamide;
(2S)-1-[5-(3-Cyclopropyl-3-fluoroazetidin-1-yl)-6-(cyclopropylmethoxy)pyridine-2-carbonyl]-4,4-difluoropyrrolidine-2-carboxamide;
N-[3-(2-Amino-2-oxoethyl)oxetan-3-yl]-5-(3-cyclopropyl-3-fluoroazetidin-1-yl)-6-(cyclopropylmethoxy)pyridine-2-carboxamide;
(2S)-1-[6-(4-Chlorophenyl)-5-(cyclopropylmethoxy)pyridine-2-carbonyl]-4,4-difluoropyrrolidine-2-carboxamide;
6-(4-Chlorophenyl)-5-(cyclopropylmethoxy)-N-[2-(5-methyl-1,2,4-oxadiazol-3-yl)propan-2-yl]pyridine-2-carboxamide;
5-(3-Cyclopropyl-3-fluoroazetidin-1-yl)-6-(cyclopropylmethoxy)-N-[3-(3-fluoropropylcarbamoyl)pentan-3-yl]pyridine-2-carboxamide; and
N-[(2S)-1-Amino-4-methyl-1-oxopentan-2-yl]-6-(4-chlorophenyl)-5-(cyclopropylmethoxy)pyridine-2-carboxamide.

11. A compound according to any one of claims 1 to 10 selected from
Methyl 2-[[5-(4-chlorophenyl)-6-(cyclopropylmethoxy)pyridine-2-carbonyl] amino]-2-methylpropanoate;
6-(Cyclopropylmethoxy)-N-[2-(1,3-oxazol-2-yl)propan-2-yl]-5-phenylpyridine-2-carboxamide;
6-(3-Chlorophenyl)-5-(cyclopropylmethoxy)-N-[2-(1,3-thiazol-2-yl)propan-2-yl]pyridine-2-carboxamide;
N-[(2S)-1-amino-4-methyl-1-oxopentan-2-yl]-6-(cyclopropylmethoxy)-5-(3-methoxyazetidin-1-yl)pyridine-2-carboxamide;
3-[6-(Cyclopropylmethoxy)-5-(3-methoxyazetidin-1-yl)pyridine-2-carbonyl]-1,1-dioxo-1,3-thiazolidine-4-carboxamide;
Ethyl 2-[[6-(cyclopropylmethoxy)-5-(3-methoxyazetidin-1-yl)pyridine-2-carbonyl] amino] -2-ethylbutanoate;
N-[3-(2-Amino-2-oxoethyl)oxetan-3-yl]-6-(cyclopropylmethoxy)-5-(3-methoxyazetidin-1-yl)pyridine-2-carboxamide;
6-(Cyclopropylmethoxy)-5-(3-methoxyazetidin-1-yl)-N-[3-(methylcarbamoyl)pentan-3-yl]pyridine-2-carboxamide; and
N-[(2S)-1-Amino-4-methyl-1-oxopentan-2-yl]-6-(4-chlorophenyl)-5-(cyclopropylmethoxy)pyridine-2-carboxamide.

12. A process for the preparation of a compound according to any one of claims 1 to 11, comprising the reaction of a compound of formula (A) 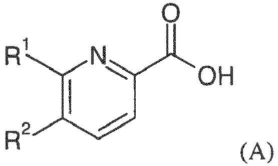 in the presence of NHR³R⁴, an amide bond forming coupling agent and a base, wherein R¹ to R⁴ are as defined in any one of claims 1 to 9.

13. A compound according to any one of claims 1 to 11 for use as therapeutically active substance.

14. A pharmaceutical composition comprising a compound in accordance with any one of claims 1 to 11 and a therapeutically inert carrier.

15. A compound according to any one of claims 1 to 11 for use in the treatment or prophylaxis of pain, neuropathic pain, asthma, osteoporosis, inflammation, psychiatric diseases, psychosis, oncology, encephalitis, malaria, allergy, immunological disorders, arthritis, gastrointestinal disorders, psychiatric disorders rheumatoid arthritis, psychosis or allergy.

## Patentansprüche

1. Verbindung der Formel (I) 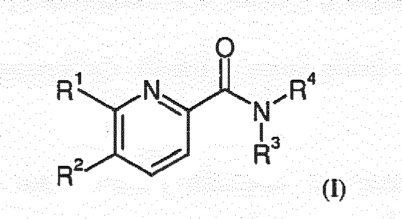 wobei
R¹ Alkylsulfanyl, Cycloalkylalkoxy, Halogenphenyl oder Halogen ist;
R² Phenyl, Halogenphenyl, Cycloalkylalkoxy, Alkoxyazetidinyl, 2-Oxa-6-azaspiro[3.3]heptyl, (Halogenalkyl)(hydroxy)pyrrolidinyl, Halogen-5-azaspiro[2.4]heptyl, (Alkyl)(halogen)azetidinyl oder (Cycloalkyl)(halogen)azetidinyl ist;
einer von R³ und R⁴ Wasserstoff ist und der andere -(CR⁵R⁶)ₘ-(CH₂)ₙ-R⁷ ist;
oder R³ und R⁴ zusammen mit dem Stickstoffatom, an das sie gebunden sind, Aminocarbonyl-dioxo-thiazolidinyl oder (Aminocarbonyl)(halogen)pyrrolidinyl bilden;
R⁵ und R⁶ unabhängig voneinander aus Wasserstoff, Alkyl, Cycloalkylalkyl und Alkylsulfanylalkyl ausgewählt sind;
oder R⁵ und R⁶ zusammen mit dem Stickstoffatom, an das sie gebunden sind, Oxetanyl bilden;
R⁷ Alkoxycarbonyl, Oxazol-2-yl, 5-Alkyl-1,2,4-oxadiazol-3-yl, Aminocarbonyl, Alkylaminocarbonyl, Thiazol-2-yl, 5-Halogenphenyl-1,3,4-oxadiazol-2-yl oder Hydroxycycloalkyl, Halogenalkylaminocarbonyl oder Halogenazetidinylcarbonyl ist;
m 0 oder 1 ist;
n 0 oder 1 ist;
oder ein pharmazeutisch unbedenkliches Salz oder ein pharmazeutisch unbedenklicher Ester davon.

2. Verbindung nach Anspruch 1, wobei R¹ Cycloalkylalkoxy oder Halogenphenyl ist.

3. Verbindung nach Anspruch 1 oder 2, wobei R¹ Cyclopropylmethoxy oder Chlorphenyl ist.

4. Verbindung nach einem der Ansprüche 1 bis 3, wobei R² Phenyl, Halogenphenyl, Cycloalkylalkoxy oder Alkoxyazetidinyl ist.

5. Verbindung nach einem der Ansprüche 1 bis 4, wobei R² Phenyl, Chlorphenyl, Cyclopropylmethoxy oder Methoxyazetidinyl ist.

6. Verbindung nach einem der Ansprüche 1 bis 5, wobei R⁵ und R⁶ unabhängig voneinander aus Wasserstoff und Alkyl ausgewählt sind.

7. Verbindung nach einem der Ansprüche 1 bis 6, wobei R⁵ und R⁶ unabhängig voneinander aus Wasserstoff, Methyl, Ethyl und Isobutyl ausgewählt sind.

8. Verbindung nach einem der Ansprüche 1 bis 7, wobei R⁷ Alkoxycarbonyl, Oxazol-2-yl, Thiazol-2-yl oder Aminocarbonyl ist.

9. Verbindung nach einem der Ansprüche 1 bis 8, wobei R⁷ Methoxycarbonyl, Oxazol-2-yl, Thiazol-2-yl oder Aminocarbonyl ist.

10. Verbindung nach einem der Ansprüche 1 bis 9, ausgewählt aus
Methyl 2-[[5-(4-chlorphenyl)-6-methylsulfanylpyridin-2-carbonyl]amino]-2-methylpropanoat;
Methyl 2-[[5-(4-chlorphenyl)-6-(cyclopropylmethoxy)pyridin-2-carbonyl]amino]-2-methylpropanoat;
5-(4-Chlorphenyl)-6-(cyclopropylmethoxy)-*N*-[2-(1,3-oxazol-2-yl)propan-2-yl]pyridin-2-carboxamid;
5-(4-Chlorphenyl)-6-(cyclopropylmethoxy)-*N*-[2-(5-methyl-1,2,4-oxadiazol-3-yl)propan-2-yl]pyridin-2-carboxamid;
6-(Cyclopropylmethoxy)-*N*-[2-(1,3-oxazol-2-yl)propan-2-yl]-5-phenylpyridin-2-carboxamid;
6-(Cyclopropylmethoxy)-N-[3-(methylcarbamoyl)pentan-3-yl]-5-phenylpyridin-2-carboxamid;
6-(3-Chlorphenyl)-5-(cyclopropylmethoxy)-*N*-[3-(methylcarbamoyl)pentan-3-yl]pyridin-2-carboxamid;
6-(Cyclopropylmethoxy)-5-(3-methoxyazetidin-1-yl)-*N*-[2-(5-methyl-1,2,4-exadiazol-3-yl)propan-2-yl]pyridin-2-carboxamid;
6-(3-Chlorphenyl)-5-(cyclopropylmethoxy)-*N*-[2-(5-methyl-1,2,4-oxadiazol-3-yl)propan-2-yl]pyridin-2-carboxamid;
*N*-[(2*S*)-1-Amino-3-cyclopropyl-1-oxopropan-2-yl]-6-(cyclopropylmethoxy)-5-(3-methoxyazetidin-1-yl)pyridin-2-carboxamid;
6-(Cyclopropylmethoxy)-5-(2-oxa-6-azaspiro[3.3]heptan-6-yl)-*N*-[2-(1,3-thiazol-2-yl)propan-2-yl]pyridin-2-carboxamid;
*N*-[(2*S*)-1-Amino-4-methyl-1-oxopentan-2-yl]-6-(cyclopropylmethoxy)-5-(2-oxa-6-azaspiro[3.3]heptan-6-yl)pyridin-2-carboxamid;
6-(3-Chlorphenyl)-5-(cyclopropylmethoxy)-*N*-[2-(1,3-thiazol-2-yl)propan-2-yl]pyridin-2-carboxamid;
*N*-[(2*S*)-1-Amino-3-cyclopropyl-1-oxopropan-2-yl]-5,6-bis(cyclopropylmethoxy)pyridin-2-carboxamid;
*N*-[(2*S*)-1-Amino-4-methyl-1-oxopentan-2-yl]-6-(cyclopropytmethoxy)-5-(3-methoxyazetidin-1-yl)pyridin-2-carboxamid;
*N*-[(2*S*)-1-Amino-4-methylsulfanyl-1-oxobutan-2-yl]-6-chlor-5-(cyclopropylmethoxy)pyridin-2-carboxamid;
6-Chlor-5-(cyclopropylmethoxy)-*N*-[3-(methylcarbamoyl)pentan-3-yl]pyridin-2-carboxamid;
6-Chlor-5-(cyclopropylmethoxy)-*N*-[(1*S*)-2-cyclopropyl-1-(5-methyl-1,2,4-oxadiazel-3-yl)ethyl]pyridin-2-carboxamid;
3-[6-(Cyclopropylmethoxy)-5-(3-methoxyazetidin-1-yl)pyridin-2-carbonyl]-1,1-dioxo-1,3-thiazolidin-4-carboxamid;
Ethyl 2-[[6-(cyclopropylmethoxy)-5-(3-methoxyazetidin-1-yl)pyridin-2-carbonyl]amino]-2-ethylbutanoat;
6-(Cyclopropylmethoxy)-*N*-[-[5-(4-fluorphenyl)-1,3,4-oxadiazol-2-yl]-2-methylpropan-2-yl]-5-(3-methoxyazetidin-1-yl)pyridin-2-carboxamid;
N-[3-(2-Amino-2-oxoethyl)oxetan-3-yl]-6-(cyclopropylmethoxy)-5-(3-methoxyazetidin-1-yl)pyridin-2-carboxamid;
6-(Cyclopropylmethoxy)-5-(3-methoxyazetidin-1-yl)-N-[3-(methylcarbamoyl)pentan-3-yl]pyridin-2-carboxamid;
6-(Cyclopropylmethoxy)-N-[(1-hydroxycyclohexyl)methyl]-5-(3-methoxyazetidin-1-yl)pyridin-2-carboxamid;
6-(Cyclopropylmethoxy)-N-[3-(2-fluorethylcarbamoyl)pentan-3-yl]-5-(3-methoxyazetidin-1-yl)pyridin-2-carboxamid;
6-(Cyclopropylmethoxy)-N-[3-(3-fluerazetidin-1-carbonyl)pentan-3-yl]-5-(3-methoxyazetidin-1-yl)pyridin-2-carboxamid;
6-(Cyclopropylmethoxy)-N-[3-(3-fluorpropylcarbamoyl)pentan-3-yl]-5-(3-methoxyazetidin-1-yl)pyridin-2-carboxamid;
(2S)-1-[6-(Cyclopropylmethoxy)-5-[3-hydroxy-3-(trifluormethyl)pyrrolidin-1-yl]pyridin-2-carbonyl]-4,4-difluorpyrrolidin-2-carboxamid;
N-[(2S)-1-Amino-4-methyl-1-oxopentan-2-yl]-6-(cyclopropylmethoxy)-5-[3-hydroxy-3-(trifluormethyl)pyrrolidin-1-yl]pyridin-2-carboxamid,
N-[(2S)-1-Amino-4-methyl-1-oxopentan-2-yl]-6-(cyclopropylmethoxy)-5-(2,2-difluor-5-azaspiro[2.4]heptan-5-yl)pyridin-2-carboxamid;
(2S)-1-[6-(Cyclopropylmethoxy)-5-(2,2-difluor-5-azaspiro[2.4]heptan-5-yl)pyridin-2-carbonyl]-4,4-difluorpyrrolidin-2-carboxamid;
N-[3-(2-Amino-2-oxoethyl)oxetan-3-yl]-6-(cyclopropylmethoxy)-5-(2,2-difluor-5-azaspiro[2.4]heptan-5-yl)pyridin-2-carboxamid;
N-[(2S)-1-Amino-4-methyl-1-oxopentan-2-yl]-6-(cyclopropylmethoxy)-5-(3-fluor-3-methylazetidin-1-yl)pyridin-2-carboxamid;
(2S)-1-[6-(Cyclopropylmethoxy)-5-(3-fluor-3-methylazetidin-1-yl)pyridin-2-carbonyl]-4,4-difluorpyrrolidin-2-carboxamid;
N-[3-(2-Amino-2-oxoethyl)oxetan-3-yl]-6-(cyclopropylmethoxy)-5-(3-fluor-3-methylazetidin-1-yl)pyridin-2-carboxamid;
N-[(2S)-1-Amino-4-methyl-1-oxopentan-2-yl]-5-(3-cyclopropyl-3-fluorazetidin-1-yl)-6-(cyclopropylmethoxy)pyridin-2-carboxamid;
(2S)-1-[5-(3-Cyclopropyl-3-fluorazetidin-1-yl)-6-(cyclopropylmethoxy)pyridin-2-carbonyl]-4,4-difluorpyrrolidin-2-carboxamid,
N-[3-(2-Amino-2-oxoethyl)oxetan-3-yl]-5-(3-cyclopropyl-3-fluorazetidin-1-yl)-6-(cyclopropylmethoxy)pyridin-2-carboxamid;
(2S)-1-[6-(4-Chlorphenyl)-5-(cyclopropylmethoxy)pyridin-2-carbonyl]-4,4-difluorpyrrolidin-2-carboxamid;
6-(4-Chlorphenyl)-5-(cyclopropylmethoxy)-N-[2-(5-methyl-1,2,4-oxadiazol-3-yl)propan-2-yl]pyridin-2-carboxamid;
5-(3-Cyclopropyl-3-fluorazetidin-1-yl)-6-(cyclopropylmethoxy)-N-[3-(3-fluorpropylcarbamoyl)pentan-3-yl]pyridin-2-carboxamid; und
N-[(2S)-1-Amino-4-methyl-1-oxopentan-2-yl]-6-(4-chlorphenyl)-5-(cyclopropylmethoxy)pyridin-2-carboxamid.

11. Verbindung nach einem der Ansprüche 1 bis 10, ausgewählt aus
Methyl 2-[[5-(4-chlorphenyl)-6-(cyclopropylmethoxy)pyridin-2-carbonyl]amino]-2-methylpropanoat;
6-(Cyclopropylmethoxy)-N-[2-(1,3-oxazol-2-yl)propan-2-yl]-5-phenylpyridin-2-carboxamid;
6-(3-Chlorphenyl)-5-(cyclopropylmethoxy)-N-[2-(1,3-thiazol-2-yl)propan-2-yl]pyridin-2-carboxamid;
N-[(2S)-1-Amino-4-methyl-1-oxopentan-2-yl]-6-(cyclopropylmethoxy)-5-(3-methoxyazetidin-1-yl)pyridin-2-carboxamid;
3-[6-(Cyclopropylmethoxy)-5-(3-methoxyazetidin-1-yl)pyridin-2-carbonyl]-1,1-dioxo-1,3-thiazolidin-4-carboxamid;
Ethyl 2-[[6-(cyclopropylmethoxy)-5-(3-methoxyazetidin-1-yl)pyridin-2-carbonyl]amino]-2-ethylbutanoat;
N-[3-(2-Amino-2-oxoethyl)oxetan-3-yl]-6-(cyclopropylmethoxy)-5-(3-methoxyazetidin-1-yl)pyridin-2-carboxamid;
6-(Cyclopropylmethoxy)-5-(3-methoxyazetidin-1-yl)-N-[3-(methylcarbamoyl)pentan-3-yl]pyridin-2-carboxamid; und
N-[(2S)-1-Amino-4-methyl-1-oxopentan-2-yl]-6-(4-chlorphenyl)-5-(cyclopropylmethoxy)pyridin-2-carboxamid.

12. Verfahren zur Herstellung einer Verbindung nach einem der Ansprüche 1 bis 11, umfassend die Reaktion einer Verbindung der Formel (A) 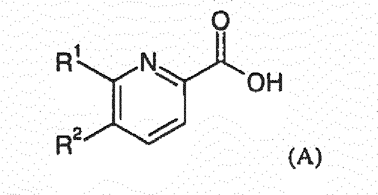 in Gegenwart von NHR³R⁴, einem Amidbindungen bildenden Kupplungsmittel und einer Base, wobei R¹ bis R⁴ wie in einem der Ansprüche 1 bis 9 definiert sind.

13. Verbindung nach einem der Ansprüche 1 bis 11 zur Verwendung als therapeutisch wirksame Substanz.

14. Pharmazeutische Zusammensetzung, umfassend eine Verbindung nach einem der Ansprüche 1 bis 11 und einen therapeutisch inerten Träger.

15. Verbindung nach einem der Ansprüche 1 bis 11 zur Verwendung bei der Behandlung oder Prophylaxe von Schmerzen, neuropathischen Schmerzen, Asthma, Osteoporose, Entzündungen, psychiatrischen Krankheiten, Psychose, Onkologie, Enzephalitis, Malaria, Allergie, immunologischen Störungen, Arthritis, gastrointestinalen Störungen, psychiatrischen Störungen rheumatoider Arthritis, Psychose oder Allergie.

## Revendications

1. Composé de formule (I) 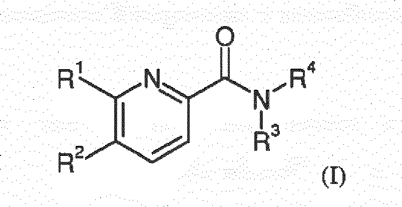 dans laquelle
R¹ représente un alkylsulfanyle, un cycloalkylalcoxy, un halogénophényle ou un halogène ;
R² représente un phényle, un halogénophényle, un cycloalkylalcoxy, un alcoxyazétidinyle, un 2-oxa-6-azaspiro[3.3]heptyle, un (halogénoalkyl)(hydroxy)pyrrolidinyle, un halogéno-5-azaspiro[2.4]heptyle, un (alkyl)(halogéno)azétidinyle ou un (cycloalkyl)(halogéno)azétidinyle ;
un parmi R³ et R⁴ représente un hydrogène et l'autre représente -(CR⁵R⁶)ₘ-(CH₂)ₙ-R⁷ ;
ou R³ et R⁴ conjointement avec l'atome d'azote auquel ils sont fixés forment un aminocarbonyl-dioxo-thiazolidinyle ou un (aminocarbonyl)(halogéno)pyrrolidinyle ;
R⁵ et R⁶ sont indépendamment choisis parmi un hydrogène, un alkyle, un cycloalkylalkyle et un alkylsulfanylalkyle ;
ou R⁵ et R⁶ conjointement avec l'atome de carbone auquel ils sont fixés forment un oxétanyle ;
R⁷ représente un alcoxycarbonyle, un oxazol-2-yle, un 5-alkyl-1,2,4-oxadiazol-3-yle, un aminocarbonyle, un alkylaminocarbonyle, un thiazol-2-yle, un 5-halogénophényl-1,3,4-oxadiazol-2-yle ou un hydroxycycloalkyle, un halogénoalkylaminocarbonyle ou un halogénoazétidinylcarbonyle ;
m représente 0 ou 1 ;
n représente 0 ou 1 ;
ou un de leurs esters ou sels pharmaceutiquement acceptables.

2. Composé selon la revendication 1, dans lequel R¹ représente un cycloalkylalcoxy ou un halogénophényle.

3. Composé selon la revendication 1 ou 2, dans lequel R¹ représente un cyclopropylméthoxy ou un chlorophényle.

4. Composé selon l'une quelconque des revendications 1 à 3, dans lequel R² représente un phényle, un halogénophényle, un cycloalkylalcoxy ou un alcoxyazétidinyle.

5. Composé selon l'une quelconque des revendications 1 à 4, dans lequel R² représente un phényle, un chlorophényle, un cyclopropylméthoxy ou un méthoxyazétidinyle.

6. Composé selon l'une quelconque des revendications 1 à 5, dans lequel R⁵ et R⁶ sont indépendamment choisis parmi un hydrogène et un alkyle.

7. Composé selon l'une quelconque des revendications 1 à 6, dans lequel R⁵ et R⁶ sont indépendamment choisis parmi un hydrogène, un méthyle, un éthyle et un isobutyle.

8. Composé selon l'une quelconque des revendications 1 à 7, dans lequel R⁷ représente un alcoxycarbonyle, un oxazol-2-yle, un thiazol-2-yle ou un aminocarbonyle.

9. Composé selon l'une quelconque des revendications 1 à 8, dans lequel R⁷ représente un méthoxycarbonyle, un oxazol-2-yle, un thiazol-2-yle ou un aminocarbonyle.

10. Composé selon l'une quelconque des revendications 1 à 9 choisi parmi
le 2-[[5-(4-chlorophényl)-6-méthylsulfanylpyridine-2-carbonyl]amino]-2-méthylpropanoate de méthyle ;
le 2-[[5-(4-chlorophényl)-6-(cyclopropylméthoxy)pyridine-2-carbonyl]amino]-2-méthylpropanoate de méthyle ;
le 5-(4-chlorophényl)-6-(cyclopropylméthoxy)-*N*-[2-(1,3-oxazol-2-yl)propan-2-yl]pyridine-2-carboxamide ;
le 5-(4-chlorophényl)-6-(cyclopropylméthoxy)-*N*-[2-(5-méthyl-1,2,4-oxadiazol-3-yl)propan-2-yl]pyridine-2-carboxamide ;
le 6-(cyclopropylméthoxy)-*N*-[2-(1,3-oxazol-2-yl)propan-2-yl]-5-phénylpyridine-2-carboxamide ;
le 6-(cyclopropylméthoxy)-*N*-[3-(méthylcarbamoyl)pentan-3-yl]-5-phénylpyridine-2-carboxamide ;
le 6-(3-chlorophényl)-5-(cyclopropylméthoxy)-*N*-[3-(méthylcarbamoyl)pentan-3-yl]pyridine-2-carboxamide ;
le 6-(cyclopropylméthoxy)-5-(3-méthoxyazétidin-1-yl)-*N*-[2-(5-méthyl-1,2,4-oxadiazol-3-yl)propan-2-yl]pyridine-2-carboxamide ;
le 6-(3-chlorophényl)-5-(cyclopropylméthoxy)-*N*-[2-(5-méthyl-1,2,4-oxadiazol-3-yl)propan-2-yl]pyridine-2-carboxamide ;
le *N*-[(2*S*)-1-amino-3-cyclopropyl-1-oxopropan-2-yl]-6-(cyclopropylméthoxy)-5-(3-méthoxyazétidin-1-yl)pyridine-2-carboxamide ;
le 6-(cyclopropylméthoxy)-5-(2-oxa-6-azaspiro[3.3]heptan-6-yl)-*N*-[2-(1,3-thiazol-2-yl)propan-2-yl]pyridine-2-carboxamide ;
le *N*-[(2*S*)-1-amino-4-méthyl-1-oxopentan-2-yl]-6-(cyclopropylméthoxy)-5-(2-oxa-6-azaspiro[3.3]heptan-6-yl)pyridine-2-carboxamide ;
le 6-(3-chlorophényl)-5-(cyclopropylméthoxy)-*N*-[2-(1,3-thiazol-2-yl)propan-2-yl]pyridine-2-carboxamide ;
le *N*-[(2*S*)-1-amino-3-cyclopropyl-1-oxopropan-2-yl]-5,6-bis(cyclopropylméthoxy)pyridine-2-carboxamide ;
le *N*-[(2*S*)-1-amino-4-méthyl-1-oxopentan-2-yl]-6-(cyclopropylméthoxy)-5-(3-méthoxyazétidin-1-yl)pyridine-2-carboxamide ;
le *N*-[(2*S*)-1-amino-4-méthylsulfanyl-1-oxobutan-2-yl]-6-chloro-5-(cyclopropylméthoxy)pyridine-2-carboxamide ;
le 6-chloro-5-(cyclopropylméthoxy)-*N*-[3-(méthylcarbamoyl)pentan-3-y]pyridine-2-carboxamide ;
le 6-chloro-5-(cyclopropylméthoxy)-*N*-[(1*S*)-2-cyclopropyl-1-(5-méthyl-1,2,4-oxadiazol-3-yl)éthyl]pyridine-2-carboxamide ;
le 3-[6-(cyclopropylméthoxy)-5-(3-méthoxyazétidin-1-yl)pyridine-2-carbonyl]-1,1-dioxo-1,3-thiazolidine-4-carboxamide ;
le 2-[[6-(cyclopropylméthoxy)-5-(3-méthoxyazétidin-1-yl)pyridine-2-carbonyl]amino]-2-éthylbutanoate d'éthyle ;
le 6-(cyclopropylméthoxy)-*N*-[1-[5-(4-fluorophényl)-1,3,4-oxadiazol-2-yl]-2-méthylpropan-2-yl]-5-(3-méthoxyazétidin-1-yl)pyridine-2-carboxamide ;
le N-[3-(2-amino-2-oxoéthyl)oxétan-3-yl]-6-(cyclopropylméthoxy)-5-(3-méthoxyazétidin-1-yl)pyridine-2-carboxamide ;
le 6-(cyclopropylméthoxy)-5-(3-méthoxyazétidin-1-yl)-N-[3-(méthylcarbamoyl)pentan-3-yl]pyridine-2-carboxamide ;
le 6-(cyclopropylméthoxy)-N-[(1-hydroxycyclohexyl)méthyl]-5-(3-méthoxyazétidin-1-yl)pyridine-2-carboxamide ;
le 6-(cyclopropylméthoxy)-N-[3-(2-fluoroéthylcarbamoyl)pentan-3-yl]-5-(3-méthoxyazétidin-1-yl)pyridine-2-carboxamide ;
le 6-(cyclopropylméthoxy)-N-[3-(3-fluoroazétidine-1-carbonyl)pentan-3-yl]-5-(3-méthoxyazétidin-1-yl)pyridine-2-carboxamide ;
le 6-(cyclopropylméthoxy)-N-[3-(3-fluoropropylcarbamoyl)pentan-3-yl]-5-(3-méthoxyazétidin-1-yl)pyridine-2-carboxamide ;
le (2S)-1-[6-(cyclopropylméthoxy)-5-[3-hydroxy-3-(trifluorométhyl)pyrrolidin-1-yl]pyridine-2-carbonyl]-4,4-difluoropyrrolidine-2-carboxamide ;
le N-[(2S)-1-amino-4-méthyl-1-oxopentan-2-yl]-6-(cyclopropylméthoxy)-5-[3-hydroxy-3-(trifluorométhyl)pyrrolidin-1-yl]pyridine-2-carboxamide ;
le N-[(2S)-1-amino-4-méthyl-1-oxopentan-2-yl]-6-(cyclopropylméthoxy)-5-(2,2-difluoro-5-azaspiro[2.4]heptan-5-yl)pyridine-2-carboxamide ;
le (2S)-1-[6-(cyclopropylméthoxy)-5-(2,2-difluoro-5-azaspiro[2.4]heptan-5-yl)pyridine-2-carbonyl]-4,4-difluoropyrrolidine-2-carboxamide ;
le N-[3-(2-amino-2-oxoéthyl)oxétan-3-yl]-6-(cyclopropylméthoxy)-5-(2,2-difluoro-5-azaspiro[2.4]heptan-5-yl)pyridine-2-carboxamide ;
le N-[(2S)-1-amino-4-méthyl-1-oxopentan-2-yl]-6-(cyclopropylméthoxy)-5-(3-fluoro-3-méthylazétidin-1-yl)pyridine-2-carboxamide ;
le (2S)-1-[6-(cyclopropylméthoxy)-5-(3-fluoro-3-méthylazétidin-1-yl)pyridine-2-carbonyl]-4,4-difluoropyrrolidine-2-carboxamide ;
le N-[3-(2-amino-2-oxoéthyl)oxétan-3-yl]-6-(cyclopropylméthoxy)-5-(3-fluoro-3-méthylazétidin-1-yl)pyridine-2-carboxamide ;
le N-[(2S)-1-amino-4-méthyl-1-oxopentan-2-yl]-5-(3-cyclopropyl-3-fluoroazétidin-1-yl)-6-(cyclopropylméthoxy)pyridine-2-carboxamide ;
le (2S)-1-[5-(3-cyclopropyl-3-fluoroazétidin-1-yl)-6-(cyclopropylméthoxy)pyridine-2-carbonyl]-4,4-difluoropyrrolidine-2-carboxamide ;
le N-[3-(2-amino-2-oxoéthyl)oxétan-3-yl]-5-(3-cyclopropyl-3-fluoroazétidin-1-yl)-6-(cyclopropylméthoxy)pyridine-2-carboxamide ;
le (2S)-1-[6-(4-chlorophényl)-5-(cyclopropylméthoxy)pyridine-2-carbonyl]4,4-difluoropyrrolidine-2-carboxamide ;
le 6-(4-chlorophényl)-5-(cyclopropylméthoxy)-N-[2-(5-méthyl-1,2,4-oxadiazol-3-yl)propan-2-yl]pyridine-2-carboxamide ;
le 5-(3-cyclopropyl-3-fluoroazétidin-1-yl)-6-(cyclopropylméthoxy)-N-[3-(3-fluoropropylcarbamoyl)pentan-3-yl]pyridine-2-carboxamide ; et
le N-[(2S)-1-amino-4-méthyl-1-oxopentan-2-yl]-6-(4-chlorophényl)-5-(cyclopropylméthoxy)pyridine-2-carboxamide.

11. Composé selon l'une quelconque des revendications 1 à 10 choisi parmi
le 2-[[5-(4-chlorophényl)-6-(cyclopropylméthoxy)pyridine-2-carbonyl]amino]-2-méthylpropanoate de méthyle ;
le 6-(cyclopropylméthoxy)-N-[2-(1,3-oxazol-2-yl)propan-2-yl]-5-phénylpyridine-2-carboxamide ;
le 6-(3-chlorophényl)-5-(cyclopropylméthoxy)-N-[2-(1,3-thiazol-2-yl)propan-2-yl]pyridine-2-carboxamide ;
le N-[(2S)-1-amino-4-méthyl-1-oxopentan-2-yl]-6-(cyclopropylméthoxy)-5-(3-méthoxyazétidin-1-yl)pyridine-2-carboxamide ;
le 3-[6-(cyclopropylméthoxy)-5-(3-méthoxyazétidin-1-yl)pyridine-2-carbonyl]-1,1-dioxo-1,3-thiazolidine-4-carboxamide ;
le 2-[[6-(cyclopropylméthoxy)-5-(3-méthoxyazétidin-1-yl)pyridine-2-carbonyl]amino]-2-éthylbutanoate d'éthyle ;
le N-[3-(2-amino-2-oxoéthyl)oxétan-3-yl]-6-(cyclopropylméthoxy)-5-(3-méthoxyazétidin-1-yl)pyridine-2-carboxamide ;
le 6-(cyclopropylméthoxy)-5-(3-méthoxyazétidin-1-yl)-N-[3-(méthylcarbamoyl)pentan-3-yl]pyridine-2-carboxamide ; et
le N-[(2S)-1-amino-4-méthyl-1-oxopentan-2-yl]-6-(4-chlorophényl)-5-(cyclopropylméthoxy)pyridine-2-carboxamide.

12. Procédé de préparation d'un composé selon l'une quelconque des revendications 1 à 11, comprenant la réaction d'un composé de formule (A) 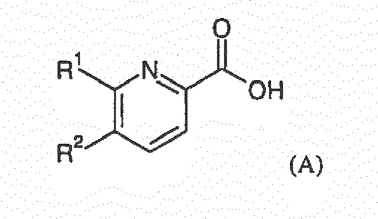 en présence de NHR³R⁴, d'un agent de couplage formant une liaison amide et d'une base, dans lesquelles R¹ à R⁴ sont tels que définis dans l'une quelconque des revendications 1 à 9.

13. Composé selon l'une quelconque des revendications 1 à 11 pour une utilisation comme substance thérapeutiquement active.

14. Composition pharmaceutique comprenant un composé selon l'une quelconque des revendications 1 à 11 et un véhicule thérapeutiquement inerte.

15. Composé selon l'une quelconque des revendications 1 à 11 pour une utilisation dans le traitement ou la prophylaxie de la douleur, de la douleur neuropathique, de l'asthme, de l'ostéoporose, de l'inflammation, des maladies psychiatriques, de la psychose, de l'oncologie, de l'encéphalite, du paludisme, de l'allergie, des troubles immunologiques, de l'arthrite, des troubles gastro-intestinaux, des troubles psychiatriques de la polyarthrite rhumatoïde, de la psychose ou de l'allergie.
